(19) Europäisches Patentamt
European Patent Office
Office européen des brevets

(11) EP 3 394 127 B1

(12) **EUROPEAN PATENT SPECIFICATION**

(45) Date of publication and mention of the grant of the patent:
**27.11.2019 Bulletin 2019/48**

(21) Application number: **16822644.7**

(22) Date of filing: **16.12.2016**

(51) Int Cl.:
*C08F 293/00* (2006.01) *C08F 230/02* (2006.01)
*A61K 8/81* (2006.01) *A61Q 1/02* (2006.01)
*A61Q 19/00* (2006.01)

(86) International application number:
**PCT/EP2016/081353**

(87) International publication number:
**WO 2017/108600 (29.06.2017 Gazette 2017/26)**

(54) **BLOCK POLYMER BEARING PHOSPHONIC ACID GROUPS AND COSMETIC USES THEREOF**

BLOCKPOLYMER MIT PHOSPHONSÄUREGRUPPEN UND KOSMETISCHE VERWENDUNGEN DAVON

POLYMER BLOC PORTANT DES GROUPES ACIDE PHOSPHONIQUE ET LEUR UTILISATIONS COSMETIQUES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **22.12.2015 FR 1563117**

(43) Date of publication of application:
**31.10.2018 Bulletin 2018/44**

(73) Proprietor: **L'Oréal**
**75008 Paris (FR)**

(72) Inventors:
- **LION, Bertrand**
  **93601 Aulnay-sous-Bois (FR)**
- **SABATIE, Laurent**
  **93601 Aulnay-sous-Bois (FR)**

(74) Representative: **L'Oreal**
**Service D.I.P.I.**
**9, rue Pierre Dreyfus**
**92110 Clichy (FR)**

(56) References cited:
**WO-A1-2009/050122 FR-A1- 2 871 470**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 394 127 B1

## Description

The present invention relates to a block polymer bearing a phosphonic acid group, to a composition comprising such a polymer and to the use of this polymer in the cosmetic field.

Block polymers, especially based on isobornyl (meth) acrylate, isobutyl acrylate and acrylic acid, which are advantageous in the field of cosmetics for their film-forming properties and their good persistence and gloss are known from patent application EP-A-1 882 709. However, it is desirable to improve the cosmetic properties of such block polymers, in particular the transfer-resistance and tack-free properties, especially when the film-forming deposit comes into contact with the fingers. It is also desirable to improve the persistence properties of the film on contact with oils or sebum.

The inventors have discovered that particular ethylenic block polymers bearing a phosphonic acid group make it possible to obtain a film that has improved persistence properties, in particular tack-free and transfer-resistance properties when the film comes into contact with the fingers.
This particular phosphonic block polymer is readily conveyable in a hydrocarbon-based oil such as isododecane.
When this phosphonic polymer is combined with an additional compound chosen from amine compounds bearing several primary amine and/or secondary amine groups, amino alkoxysilanes, salts of divalent or trivalent metal ions, clays and metal oxides, it forms a film-forming deposit that has good water-resistance, oil-resistance (especially resistance to olive oil) and sebum-resistance properties. The film also has the property of not being tacky and of not transferring on contact with a finger. The deposit obtained thus has good persistence properties.
These good film-forming properties are also obtained when the polymer is combined with a non-volatile oil (often used in makeup products).

This phosphonic block polymer combined with said additional compound forms a film-forming deposit that is suitable for making up the skin or the lips or the eyelashes, such as foundations, lipsticks or mascaras, or for fixing the hair.

More specifically, one subject of the present invention is a block polymer comprising:

at least one first block with a glass transition temperature (Tg) of greater than or equal to 40°C and obtained from at least one (meth)acrylate monomer of formula $CH_2 = C(R_1)\text{-}COOR_2$ in which $R_1$ represents H or a methyl radical and $R_2$ represents a $C_4$ to $C_{12}$ cycloalkyl group; and

at least one second block with a glass transition temperature (Tg) of less than or equal to 20°C and is obtained from at least one vinylphosphonic acid monomer of formula (I) defined below and from at least one (meth)acrylate monomer of formula $CH_2 = C(R_1)\text{-}COOR_3$ in which $R_1$ represents H or a methyl radical and $R_3$ represents either a linear or branched $C_1$ to $C_6$ unsubstituted alkyl group, with the exception of a tert-butyl group or a methoxyethyl group.

Such a block polymer is referred to hereinbelow as a phosphonic polymer.

A subject of the invention is also a composition comprising, in a physiologically acceptable medium, a phosphonic polymer as described previously.

A subject of the invention is also a process, especially a cosmetic process, for treating and in particular for caring for or making up keratin materials, comprising the topical application to the keratin materials of a composition, especially a cosmetic composition, comprising a phosphonic polymer as described previously.

A subject of the invention is in particular a process, especially a cosmetic process, for treating and in particular for caring for or making up the skin or the lips, comprising the topical application to the skin or the lips of a composition, especially a cosmetic composition, comprising a phosphonic polymer as described previously.

According to one embodiment of the process according to the invention, the phosphonic polymer used consists of the monomers described hereinabove or hereinbelow, in the described contents.

According to one embodiment of the process according to the invention, a composition obtained by mixing (extemporaneous), a composition comprising a phosphonic polymer as described previously and an additional component chosen from polyamine compounds bearing several primary amine and/or secondary amine groups, amino alkoxysilanes, salts of divalent or trivalent metal ions, clays and metal oxides, or a composition containing same and comprising a physiologically acceptable medium, is applied topically to keratin materials, the composition(s) used being anhydrous when the additional component is an amino alkoxysilane.

According to one embodiment of the process according to the invention, the mixing of the composition comprising the phosphonic polymer and of the additional component, or of the composition containing same, is performed in a time of between 1 minute and 24 hours before its application to keratin fibres, and preferably between 5 and 30 minutes.

According to a first embodiment of the process according to the invention, a composition derived from the mixing (extemporaneous) of a composition comprising a phosphonic polymer as described previously and an additional component as defined below, or of a composition containing same and comprising a physiologically acceptable medium, as defined below, is applied topically to keratin materials.

According to a second embodiment of the process according to the invention, a composition comprising a

phosphonic polymer as described previously and an additional component as defined below, or a composition containing same and comprising a physiologically acceptable medium, as defined below, are applied sequentially to keratin materials.

A subject of the invention is also a composition, especially a cosmetic composition, obtained by mixing a composition comprising, in a physiologically acceptable medium, said phosphonic polymer and an additional component as defined previously or a composition containing same and comprising a physiologically acceptable medium, the composition being anhydrous when the additional compound is an amino alkoxysilane.

A subject of the invention is also a kit comprising a first composition comprising said phosphonic polymer as described previously and a second composition comprising an additional component as defined previously and comprising a physiologically acceptable medium, the first and second compositions each being packaged in a separate packaging assembly, the compositions being anhydrous when the additional compound is an amino alkoxysilane.
The composition packaging assembly is, in a known manner, any packaging that is suitable for storing cosmetic compositions (especially a bottle, tube, spray bottle or aerosol bottle).
Such a kit makes it possible to carry out the process for treating keratin materials according to the invention.

The process according to the invention is suitable for caring for or making up keratin materials, such as the skin, the lips, the eyelashes or the nails.

The block polymer according to the invention comprises:

at least one first block with a glass transition temperature (Tg) of greater than or equal to 40°C and obtained from at least one (meth)acrylate monomer of formula $CH_2 = C(R_1)\text{-}COOR_2$ in which $R_1$ represents H or a methyl radical, and $R_2$ represents a $C_4$ to $C_{12}$ cycloalkyl group; and
at least one second block with a glass transition temperature (Tg) of less than or equal to 20°C and is obtained from at least one vinylphosphonic acid monomer of formula (I) defined below and from at least one (meth)acrylate monomer of formula $CH_2 = C(R_1)\text{-}COOR_3$ in which $R_1$ represents H or a methyl radical and $R_3$ represents a linear or branched $C_1$ to $C_6$ unsubstituted alkyl group, with the exception of a tert-butyl group.

The glass transition temperatures indicated for the first and second blocks may be theoretical Tg values determined from the theoretical Tg values of the constituent monomers of each of the blocks, which may be found in a reference manual such as the Polymer Handbook, 3rd Edition, 1989, John Wiley, according to the following relationship, known as Fox's law:

$$1/Tg = \sum_i (\omega_i / Tg_i),$$

$\omega_i$ being the mass fraction of the monomer i in the block under consideration and $Tg_i$ being the glass transition temperature of the homopolymer of the monomer i.
Unless otherwise indicated, the Tg values indicated for the first and second blocks in the present patent application are theoretical Tg values.
The difference between the glass transition temperatures of the first and second blocks is generally greater than 20°C, preferably greater than 40°C and better still greater than 60°C.

In the present invention, the expression:

"between ... and ..." means a range of values in which the limits mentioned are excluded, and
"from ... to ..." and "ranging from ... to ..." means a range of values in which the limits are included.

The block polymer used according to the invention has a first block with a glass transition temperature (Tg) of greater than or equal to 40°C, for example a Tg ranging from 40 to 150°C, and obtained from at least one (meth)acrylate monomer of formula $CH_2 = C(R_1)\text{-}COOR_2$ in which $R_1$ represents H or a methyl radical, and $R_2$ represents a $C_4$ to $C_{12}$ cycloalkyl group.

Preferably, said first block has a Tg of greater than or equal to 60°C, ranging, for example, from 60°C to 140°C, especially ranging from 80°C to 120°C.

The monomers present in the first block of the polymer and the proportions thereof are preferably chosen such that the glass transition temperature of the first block is greater than or equal to 40°C, and especially in accordance with that described previously.

According to a preferred embodiment, the first block of the polymer is obtained from at least one acrylate monomer of formula $CH_2 = CH\text{-}COOR_2$ in which $R_2$ represents a $C_4$ to $C_{12}$ cycloalkyl group, and from at least one methacrylate monomer of formula $CH_2 = C(CH_3)\text{-}COOR'_2$ in which $R'_2$ represents a $C_4$ to $C_{12}$ cycloalkyl group.

The first block of the polymer may be obtained exclusively with said acrylate monomer and said methacrylate monomer.

The acrylate monomer and the methacrylate monomer are preferably used in acrylate/methacrylate mass proportions of between 30/70 and 70/30, preferably between 40/60 and 60/40 and in particular between 45/55 and 55/45.

The proportion of the first block in the block polymer advantageously ranges from 60% to 80% and better still from 65% to 75% by weight of the polymer.

According to a preferred embodiment, the first block of the polymer is obtained by polymerization of isobornyl methacrylate and isobornyl acrylate.

The first block of the polymer may also comprise an additional monomer chosen from linear or branched C8-C22 alkyl (meth)acrylates (i.e. comprising a C8-C22 alkyl group), for instance 2-ethylhexyl acrylate, 2-ethylhexyl methacrylate, lauryl acrylate, lauryl methacrylate, behenyl acrylate, behenyl methacrylate, stearyl acrylate and stearyl methacrylate.

Said additional monomer may be present in a content ranging from 0.1% to 15% by weight and preferably ranging from 0.1% to 5% by weight, relative to the total weight of the monomers of the first block of said block polymer.

According to one embodiment, the first block of said block polymer does not contain any additional monomer.

The block polymer used according to the invention has a second block with a glass transition temperature (Tg) of less than or equal to 20°C, for example a Tg ranging from -100 to 20°C, and is obtained from at least one vinylphosphonic acid monomer of formula (I) defined below and from at least one (meth)acrylate monomer of formula $CH_2=C(R_1)-COOR_3$ in which $R_1$ represents H or a methyl radical and $R_3$ represents a linear or branched $C_1$ to $C_6$ unsubstituted alkyl group, with the exception of a tert-butyl group or a methoxyethyl group.

Preferably, said second block has a Tg of less than or equal to 10°C, especially ranging from -80°C to 15°C and better still less than or equal to 0°C, for example ranging from -100°C to 0°C, especially ranging from -30°C to 0°C.

The monomers present in the second block of the polymer and the proportions thereof are preferably chosen such that the glass transition temperature of the second block is less than or equal to 20°C, and especially in accordance with that described previously.

The vinylphosphonic acid monomer corresponds to formula (I) below:

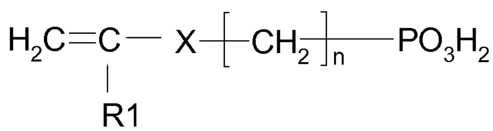

(I)

in which:

R1 denotes H or-CH3;
X denotes a covalent bond and n denotes an integer ranging from 0 to 14;
or X denotes a -COO- group and n denotes an integer ranging from 2 to 6.

Advantageously, for the monomer of formula (I), X denotes a covalent bond and n is an integer ranging from 0 to 6 or X denotes a -COO- group and n is an integer ranging from 2 to 4.

Preferentially, for the monomer of formula (I):

R1 = H

X denotes a covalent bond and n is an integer ranging from 0 to 4.

As examples of monomer of formula (I), mention may be made of:

vinylphosphonic acid;
3-butenylphosphonic acid;
4-pentenylphosphonic acid;
10-undecenylphosphonic acid;
11-dodecenylphosphonic acid;
2-phosphonoethyl ester of 2-propenoic acid;
2-phosphonoethyl ester of 2-methyl-2-propenoic acid.

Preferentially, the monomer (I) is vinylphosphonic acid.

The preferred monomers with a Tg of less than or equal to 20°C are isobutyl acrylate, ethyl acrylate, n-butyl

acrylate and methoxyethyl acrylate, or mixtures thereof in all proportions.

The second block of the polymer may be obtained exclusively with the vinylphosphonic acid monomer (I) and said acrylate monomer.

The vinylphosphonic acid monomer (I) and the acrylate monomer are preferably used in acrylate/vinylphosphonic acid monomer (I) mass proportions ranging from 1 to 10, preferentially ranging from 2 to 9, especially ranging from 3 to 8 or alternatively ranging from 4 to 7.

The proportion of the second block in the block polymer advantageously ranges from 20% to 40% and better still from 25% to 35% by weight of the polymer.

According to a preferred embodiment, the second block of the polymer is obtained by polymerization of vinylphosphonic acid and isobutyl acrylate.

The second block of the polymer may also comprise an additional silicone monomer of formula (II) (referred to hereinbelow as a silicone monomer) below:

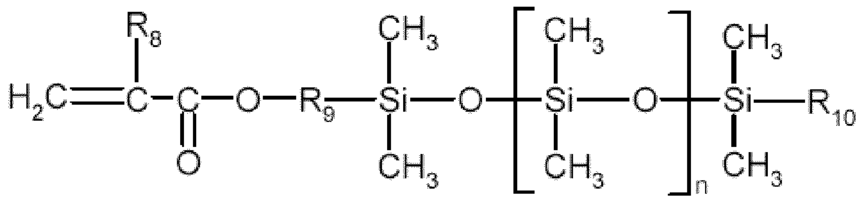

(II)

in which:

- R8 denotes a hydrogen atom or a methyl group; preferably methyl;
- R9 denotes a linear or branched, preferably linear, divalent hydrocarbon-based group containing from 1 to 10 carbon atoms, preferably containing from 2 to 4 carbon atoms, and optionally containing one or two -O- ether bonds; preferably an ethylene, propylene or butylene group;
- R10 denotes a linear or branched alkyl group containing from 1 to 10 carbon atoms, especially from 2 to 8 carbon atoms; preferably methyl, ethyl, propyl, butyl or pentyl;
- n denotes an integer ranging from 1 to 300, preferably ranging from 3 to 200 and preferentially ranging from 5 to 100.

Monomer (II) is a polydimethylsiloxane bearing a mono(meth)acryloyloxy end group.

Use may be made in particular of monomethacryloyloxypropyl polydimethylsiloxanes such as those sold under the names MCR-M07, MCR-M17, MCR-M11 and MCR-M22 by Gelest Inc or the silicone macromonomers sold under the names X-22-2475, X-22-2426 and X-22-174DX by Shin-Etsu.

Monomer (II) may be present in the second block of the block polymer in a content ranging from 0.1% to 15% by weight, relative to the total weight of the monomers of the second block of said block polymer, and preferably ranging from 0.1% to 5%.

According to one embodiment, the second block of said block polymer does not contain any additional monomer.

Preferably, the polymer used according to the invention comprises at least, or even consists of, isobornyl acrylate and isobornyl methacrylate monomers in the first block and vinylphosphonic acid and isobutyl acrylate monomers in the second block.

Preferably, the polymer comprises at least, or even consists of, isobornyl acrylate and isobornyl methacrylate monomers in a mass proportion ranging from 30/70 to 70/30 in the first block and isobutyl acrylate and vinylphosphonic acid monomers in the second block.

Preferably, the polymer comprises at least, or even consists of, isobornyl acrylate and isobornyl methacrylate monomers in a mass proportion ranging from 40/60 to 60/40 in the first block and isobutyl acrylate and vinylphosphonic acid monomers in the second block.

Preferably, the polymer comprises at least, or even consists of, isobornyl acrylate and isobornyl methacrylate monomers in a mass proportion ranging from 45/55 to 55/45 in the first block and isobutyl acrylate and vinylphosphonic acid monomers in the second block.

Preferably, the polymer comprises at least, or even consists of, isobornyl acrylate and isobornyl methacrylate monomers in a mass proportion ranging from 30/70 to 70/30 in the first block and isobutyl acrylate and vinylphosphonic acid monomers in the second block, the first block representing between 65% and 75% by weight of the polymer, and especially 70% by weight.

Preferably, the polymer comprises at least, or even consists of, isobornyl acrylate and isobornyl methacrylate monomers in a mass proportion ranging from 40/60 to 60/40 in the first block and isobutyl acrylate and vinylphosphonic acid monomers in the second block, the first block representing between 65% and 75% by weight of the polymer, and

especially 70% by weight.

Preferably, the polymer comprises at least, or even consists of, isobornyl acrylate and isobornyl methacrylate monomers in a mass proportion ranging from 45/55 to 55/45 in the first block and isobutyl acrylate and vinylphosphonic acid monomers in the second block, the first block representing between 65% and 75% by weight of the polymer, and especially 70% by weight.

Preferably, the polymer comprises at least, or even consists of, isobornyl acrylate and isobornyl methacrylate monomers in a mass proportion ranging from 30/70 to 70/30 in the first block and isobutyl acrylate and vinylphosphonic acid monomers in the second block, the first block representing between 65% and 75% by weight of the polymer, and especially 70% by weight, and the vinylphosphonic acid representing from 3% to 7% by weight of the polymer.

Preferably, the polymer comprises at least, or even consists of, isobornyl acrylate and isobornyl methacrylate monomers in a mass proportion ranging from 40/60 to 60/40 in the first block and isobutyl acrylate and vinylphosphonic acid monomers in the second block, the first block representing between 65% and 75% by weight of the polymer, and especially 70% by weight, and the vinylphosphonic acid representing from 3% to 7% by weight of the polymer.

Preferably, the polymer comprises at least, or even consists of, isobornyl acrylate and isobornyl methacrylate monomers in a mass proportion ranging from 45/55 to 55/45 in the first block and isobutyl acrylate and vinylphosphonic acid monomers in the second block, the first block representing between 65% and 75% by weight of the polymer, and especially 70% by weight, and the vinylphosphonic acid representing from 3% to 7% by weight of the polymer.

Said first and second blocks of the polymer may be advantageously linked together via an intermediate segment comprising at least one constituent monomer of the first block and at least one constituent monomer of the second block. The intermediate segment is a block comprising at least one constituent monomer of the first block and at least one constituent monomer of the second block of the polymer, which enables these blocks to be "compatibilized".
Advantageously, the intermediate segment comprising at least one constituent monomer of the first block and at least one constituent monomer of the second block of the polymer is a statistical polymer.
Preferably, the intermediate block is derived essentially from constituent monomers of the first block and of the second block.
The term "essentially" means at least 85%, preferably at least 90%, better still 95% and even better still 100%.
Advantageously, the intermediate block has a glass transition temperature Tg that is between the glass transition temperatures of the first and second blocks.

The block polymer used according to the invention is advantageously a film-forming polymer. The term "film-forming polymer" means a polymer that is capable of forming, by itself or in the presence of a film-forming auxiliary agent, a continuous film that adheres to a support, especially to keratin materials.

The polydispersity index of the block polymer is advantageously greater than 2.
The polydispersity index I of the polymer is equal to the ratio of the weight-average mass Mw to the number-average mass Mn.
The weight-average molar mass (Mw) and number-average molar mass (Mn) are determined by gel permeation liquid chromatography (THF solvent, calibration curve established with linear polystyrene standards, refractometric detector).
The weight-average mass (Mw) of the block polymer is preferably less than or equal to 300 000; it ranges, for example, from 35 000 to 200 000 and better still from 45 000 to 150 000 g/mol.
The number-average mass (Mn) of the block polymer is preferably less than or equal to 70 000; it ranges, for example, from 10 000 to 60 000 and better still from 12 000 to 50 000 g/mol.
Preferably, the polydispersity index of the block polymer is greater than 2, for example ranging from 3 to 20, preferably greater than or equal to 4, for example ranging from 4 to 18.

A subject of the invention is also a process for preparing a block polymer, which consists in mixing, in the same reactor, a polymerization solvent, an initiator, a vinylphosphonic acid monomer of formula (I) as described previously, at least one (meth)acrylate monomer of formula $CH_2=C(R_1)-COOR_3$ in which $R_1$ represents H or a methyl radical and $R_3$ represents a linear or branched $C_1$ to $C_6$ unsubstituted alkyl group, with the exception of a tert-butyl group, at least one (meth)acrylate monomer of formula $CH_2=C(R_1)-COOR_2$ in which $R_1$ represents H or a methyl radical and $R_2$ represents a $C_4$ to $C_{12}$ cycloalkyl group, according to the following sequence of steps:

- some of the polymerization solvent and some of the initiator are poured into the reactor, and the mixture is heated to a reaction temperature of between 60°C and 120°C,
- said at least one (meth)acrylate monomer of formula $CH_2=C(R_1)-COOR_2$ is then poured in, as a first addition, and the mixture is left to react for a time T corresponding to a maximum degree of conversion of said monomers of 90%,
- further polymerization initiator, the vinylphosphonic acid monomer (I) and said (meth)acrylate of formula $CH_2=C(R_1)-COOR_3$ are then poured into the reactor, as a second addition, and the mixture is left to react for a time T' after which the degree of conversion of said monomers reaches a plateau,
- the reaction mixture is cooled to room temperature.

The term "polymerization solvent" means a solvent or a mixture of solvents. The polymerization solvent may be chosen especially from ethyl acetate, butyl acetate, $C_8$-$C_{16}$ branched alkanes such as $C_8$-$C_{16}$ isoalkanes, for instance isododecane, isodecane or isohexadecane, and mixtures thereof. Preferably, the polymerization solvent is isododecane.

According to another embodiment, a subject of the invention is a process for preparing a polymer, which consists in mixing, in the same reactor, a polymerization solvent, an initiator, a vinylphosphonic acid monomer (I) as described previously, at least one (meth)acrylate monomer of formula $CH_2=C(R_1)-COOR_3$ in which $R_1$ represents H or a methyl radical and $R_3$ represents a linear or branched $C_1$ to $C_6$ unsubstituted alkyl group in which $R_3$ represents a linear or branched $C_1$ to $C_{12}$ unsubstituted alkyl group, with the exception of a tert-butyl group, at least one (meth)acrylate monomer of formula $CH_2=C(R_1)-COOR_2$ in which $R_1$ represents H or a methyl radical and $R_2$ represents a $C_4$ to $C_{12}$ cycloalkyl group in which $R_2$ represents a $C_4$ to $C_{12}$ cycloalkyl group, according to the following sequence of steps:

- some of the polymerization solvent and some of the initiator are poured into the reactor, and the mixture is heated to a reaction temperature of between 60°C and 120°C,
- the vinylphosphonic acid monomer (I) and said (meth)acrylate of formula $CH_2=C(R_1)-COOR_3$ are then poured in, as a first addition, and the mixture is left to react for a time T corresponding to a maximum degree of conversion of said monomers of 90%,
- further polymerization initiator and said at least one (meth)acrylate monomer of formula $CH_2=C(R_1)-COOR_2$ are then poured into the reactor, as a second addition, and the mixture is left to react for a time T' after which the degree of conversion of said monomers reaches a plateau,
- the reaction mixture is cooled to room temperature.

The polymerization temperature is preferably between 85 and 95°C, especially about 90°C.

The reaction time after the second addition is preferably between 3 and 6 hours.

The monomers used in the context of this process, and the proportions thereof, may be those described previously.

The polymerization is especially performed in the presence of a radical initiator especially of peroxide type (for example tert-butyl peroxy-2-ethylhexanoate: Trigonox 21S; 2,5-dimethyl-2,5-bis(2-ethylhexanoylperoxy)hexane:Trigonox 141; tert-butyl peroxypivalate: Trigonox 25C75 from AkzoNobel) or of azo type, for example (AIBN: azobisisobutyronitrile; V50: 2,2'-azobis(2-amidinopropane) dihydrochloride).

The polymer used according to the invention may be used in a composition comprising a physiologically acceptable medium, in particular in a cosmetic composition.

The term "physiologically acceptable medium" means a medium that is compatible with human keratin materials, in particular with the skin, the lips, the nails and the eyelashes.

The term "cosmetic composition" is understood to mean a composition that is compatible with keratin materials, which has a pleasant colour, odour and feel and which does not cause unacceptable discomfort (stinging, tautness or redness) liable to discourage the consumer from using it.

The phosphonic polymer as defined previously may be present in the composition according to the invention in a content ranging from 0.1% to 40% by weight, relative to the total weight of the composition derived from the extemporaneous mixing, preferably from 0.5% to 35% by weight, preferentially ranging from 1% to 30% by weight and more preferentially ranging from 10% to 30% by weight. This is the composition that is applied to the keratin materials.

The additional component used in the process according to the invention is especially an amine compound chosen from polyamine compounds containing several primary amine groups and/or secondary amine groups or alternatively amino alkoxysilanes. It may thus be chosen from amino alkoxysilane compounds, diamine compounds and triamine compounds.

According to a first embodiment of the invention, the polyamine compound is a compound comprising from 2 to 20 carbon atoms, in particular a non-polymeric compound. The term "non-polymeric compound" means a compound which is not directly obtained via a monomer polymerization reaction.

Polyamine compounds that may be mentioned include N-methyl-1,3-diaminopropane, N-propyl-1,3-diaminopropane, N-isopropyl-1,3-diaminopropane, N-cyclohexyl-1,3-diaminopropane, 2-(3-aminopropylamino)ethanol, 3-(2-aminoethyl)aminopropylamine, bis(3-aminopropyl)amine, methylbis(3-aminopropyl)amine, N-(3-aminopropyl)-1,4-diaminobutane, N,N-dimethyldipropylenetriamine, 1,2-bis(3-aminopropylamino)ethane, N,N'-bis(3-aminopropyl)-1,3-propanediamine, ethylenediamine, 1,3-propylenediamine, 1,4-butylenediamine, lysine, cystamine, xylenediamine, tris(2-aminoethyl)amine and spermidine. Preferably, the amine compound is chosen from ethylenediamine, 1,3-propylenediamine and 1,4-butylenediamine. Preferentially, the polyamine compound is ethylenediamine.

The amine compound may also be chosen from amino alkoxysilanes, such as those of formula (III):

$$R'_1Si(OR'_2)_z(R'_3)_x \qquad (III)$$

in which:

- $R'_1$ is a linear or branched, saturated or unsaturated, cyclic or acyclic $C_1$-$C_6$ hydrocarbon-based chain substituted with a group chosen from the following groups:
    - amine $NH_2$ or NHR with R = $C_1$-$C_4$ alkyl,
    - an aryl or aryloxy group substituted with an amino group or with a $C_1$-$C_4$ aminoalkyl group,
    $R'_1$ possibly being interrupted in its chain with a heteroatom (O, S, NH) or a carbonyl group (CO), R'1 being linked to the silicon atom directly via a carbon atom,
- $R'_2$ and $R'_3$, which may be identical or different, represent a linear or branched alkyl group comprising from 1 to 6 carbon atoms,
- z denotes an integer ranging from 1 to 3, and
- x denotes an integer ranging from 0 to 2,

with z + x = 3.

Preferably, $R'_2$ represents an alkyl group comprising from 1 to 4 carbon atoms.
Preferably, $R'_2$ represents a linear alkyl group comprising from 1 to 4 carbon atoms.
Preferably, $R'_2$ represents an ethyl group.

Preferably, $R'_3$ represents an alkyl group comprising from 1 to 4 carbon atoms.
Preferably, $R'_3$ represents a linear alkyl group comprising from 1 to 4 carbon atoms.
Preferably, $R'_3$ represents a methyl or ethyl group.

Preferably, $R'_1$ is an acyclic chain.

Preferably, R'1 is a linear or branched, saturated or unsaturated C1-C6 hydrocarbon-based chain, substituted with an amine group NH2 or NHR (R = C1-C6 alkyl, C3-C6 cycloalkyl or C6 aromatic). Preferentially, $R'_1$ is a saturated linear C1-C6 hydrocarbon-based chain substituted with an amine group NH2. More preferentially, $R'_1$ is a saturated linear C2-C4 hydrocarbon-based chain substituted with an amine group NH2.

Preferably, $R'_1$ is a saturated linear C1-C6 hydrocarbon-based chain substituted with an amine group NH2.
$R'_2$ represents an alkyl group comprising from 1 to 4 carbon atoms,
$R'_3$ represents an alkyl group comprising from 1 to 4 carbon atoms.

Preferably, z is equal to 3.

Preferably, the aminosilane of formula (III) is chosen from 3-aminopropyltriethoxysilane (APTES), 3-aminoethyltriethoxysilane (AETES), 3-aminopropylmethyldiethoxysilane, N-(2-aminoethyl)-3-aminopropyltriethoxysilane, 3-(m-aminophenoxy)propyltrimethoxysilane, p-aminophenyltrimethoxysilane and N-(2-aminoethylaminomethyl)phenethyltrimethoxysilane.
Preferably, the aminosilane (III) is chosen from 3-aminopropyltriethoxysilane (APTES), 3-aminoethyltriethoxysilane (AETES), 3-aminopropylmethyldiethoxysilane and N-(2-aminoethyl)-3-aminopropyltriethoxysilane.
Preferably, the aminosilane (III) is 3-aminopropyltriethoxysilane (APTES).

Preferably, the amine compound is chosen from 3-aminopropyltriethoxysilane (APTES), N-methyl-1,3-diaminopropane, N-propyl-1,3-diaminopropane, N-isopropyl-1,3-diaminopropane, N-cyclohexyl-1,3-diaminopropane, 2-(3-aminopropylamino)ethanol, 3-(2-aminoethyl)aminopropylamine, bis(3-aminopropyl)amine, methylbis(3-aminopropyl)amine, N-(3-aminopropyl)-1,4-diaminobutane, N,N-dimethyldipropylenetriamine, 1,2-bis(3-aminopropylamino)ethane, N,N'-bis(3-aminopropyl)-1,3-propanediamine, ethylenediamine and lysine.

The polyamine compound may also be chosen from amine-based polymers, especially having a weight-average molecular weight ranging from 500 to 1 000 000, preferably ranging from 500 to 500 000, and preferentially ranging from 500 to 100 000.

As amine-based polymer, use may be made of poly(($C_2$-$C_5$)alkyleneimines), and in particular polyethyleneimines and polypropyleneimines, especially poly(ethyleneimine)s (for example the product sold under the reference 46,852-3 by the company Aldrich Chemical); poly(allylamine) (for example the product sold under the reference 47,913-6 by the company Aldrich Chemical); polyvinylamines and copolymers thereof, in particular with vinylamides; mention may in particular be made of vinylamine/vinylformamide copolymers such as those sold under the name Lupamin® 9030 by the company BASF; polyamino acids bearing NH2 groups, such as polylysine, for example the product sold by the company JNC Corporation (formerly Chisso); aminodextran, such as the product sold by the company CarboMer Inc; amino polyvinyl alcohol, such as the product sold by the company CarboMer Inc, acrylamidopropylamine-based copolymers; chitosans; polydimethylsiloxanes comprising primary amine groups at the chain end or on side chains, for example aminopropyl side or end groups, for instance those of formula (A) or (B) or (C):

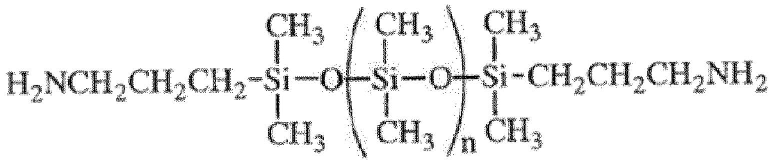

(A)

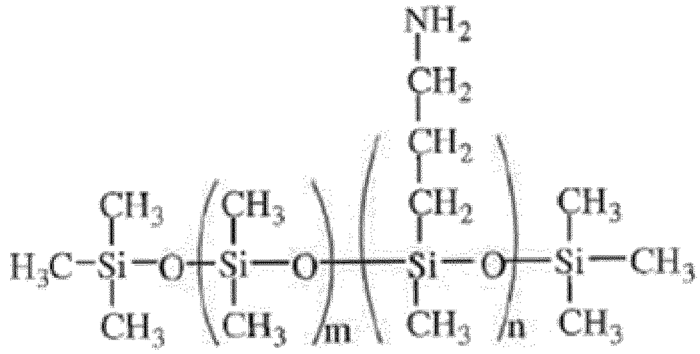

(B)

H2NCH2CH2CH2-Si(CH3)2-O-[Si(CH3)2-O]n-Si(CH3)2C4H9 (C)

in formula (A): the value of n is such that the weight-average molecular weight of the silicone is between 500 and 55 000. As examples of aminosilicone (A), mention may be made of those sold under the names DMS-A11, DMS-A12, DMS-A15, DMS-A21, DMS-A31, DMS-A32 and DMS-A35 by the company Gelest, reference 481688 from Aldrich,

in formula (B), the values of n and m are such that the weight-average molecular weight of the silicone is between 1000 and 55 000. As examples of silicone (B), mention may be made of those sold under the names AMS-132, AMS-152, AMS-162, AMS-163, AMS-191 and AMS-1203 by the company Gelest,

in formula (C), the value of n is such that the weight-average molecular weight of the silicone is between 500 and 3000. As examples of silicone (C), mention may be made of those sold under the names MCR-A11 and MCR-A12 by the company Gelest,

the amodimethicones of formula (D):

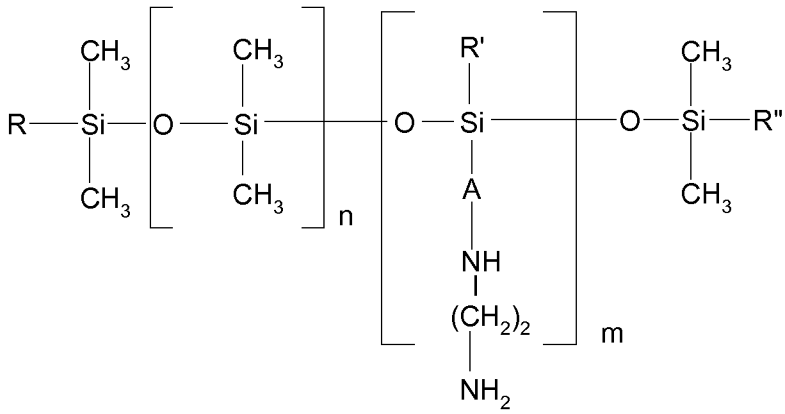

(D)

in which R, R' and R", which may be identical or different, each represent a $C_1$-$C_4$ alkyl or hydroxyl group, A represents a $C_3$ alkylene group and m and n are such that the weight-average molecular mass of the compound is between 5000 and 500 000 approximately,

the amodimethicones of formula (K):

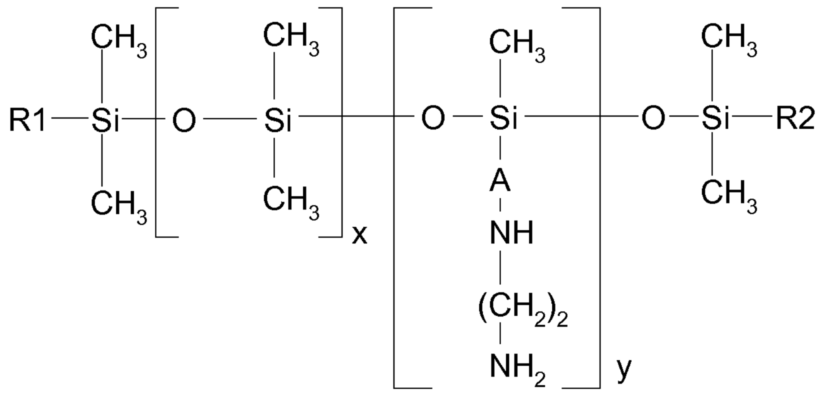

(K)

in which:

- R1 and R2, which may be identical or different, preferably identical, represent a linear or branched, saturated or unsaturated alkyl group comprising from 6 to 30 carbon atoms, preferably from 8 to 24 carbon atoms and preferentially from 12 to 20 carbon atoms,
- A represents a linear or branched alkylene radical group containing from 2 to 8 carbon atoms,
- x and y are numbers ranging from 1 to 5000; preferably, x ranges from 10 to 2000 and especially from 100 to 1000; preferably, y ranges from 1 to 100.

Preferably, A comprises from 3 to 6 carbon atoms, in particular 4 carbon atoms; preferably, A is branched. A may be a divalent radical chosen from: $-CH_2CH_2CH_2-$ and $-CH_2CH(CH_3)CH_2-$.

Preferably, R1 and R2, which may be identical or different, represent a saturated linear alkyl group comprising from 6 to 30 carbon atoms, preferentially from 8 to 24 carbon atoms and especially from 12 to 20 carbon atoms, for instance a dodecyl, tetradecyl, pentadecyl, hexadecyl, heptadecyl, octadecyl, nonadecyl or eicosyl group. Advantageously, R1 and R2 represent a mixture of hexadecyl (cetyl) and octadecyl (stearyl) radicals (mixture also known as cetearyl).

Preferentially, for the amodimethicone of formula (K):

- x ranges from 10 to 2000 and especially from 100 to 1000;
- y ranges from 1 to 100;
- A comprises from 3 to 6 carbon atoms, and in particular 4 carbon atoms; preferably, A is branched; preferentially, A is chosen from the divalent radicals: $-CH_2CH_2CH_2-$ and
- $CH_2CH(CH_3)CH_2-$; and
- R1 and R2, which may be identical or different, represent a saturated linear radical comprising from 6 to 30 carbon atoms, preferably from 8 to 24 carbon atoms and especially from 12 to 20 carbon atoms, for instance a dodecyl, tetradecyl, pentadecyl, hexadecyl, heptadecyl, octadecyl, nonadecyl or eicosyl group. Advantageously, R1 and R2 represent a mixture of hexadecyl (cetyl) and octadecyl (stearyl) radicals (mixture also known as cetearyl);

As amodimethicone of formula (K), use may be made of bis-cetearyl amodimethicone (INCI name), especially the product sold under the name Silsoft® AX by the company Momentive Performance Materials.

The polyether amines known especially under the reference Jeffamine from the company Huntsman; and especially:

polyethylene glycol and/or polypropylene glycol $\alpha,\omega$-diamines (bearing an amine function at the end of the chain), which may comprise from 2 to 80 units derived from propylene oxide, or which may comprise from 2 to 50 units derived from ethylene oxide and from 1 to 10 units derived from propylene oxide, for instance the products sold under the names Jeffamine D-230, D-400, D-2000, D-4000, ED-600, ED-9000 and ED-2003;

polytetrahydrofuran (or polytetramethylene glycol) $\alpha,\omega$-diamines;

polybutadiene $\alpha,\omega$-diamines;

polyamidoamine (PANAM) dendrimers bearing amine end functions;

poly(meth)acrylates or poly(meth)acrylamides bearing primary or secondary amine side functions, such as poly(3-aminopropyl)methacrylamide or poly(2-aminoethyl) methacrylate.

As amine-based polymer, use is preferably made of polydimethylsiloxanes comprising primary amine groups

at the chain end or on side chains.
Preferentially, polydimethylsiloxanes comprising aminopropyl end groups at the chain end are used.

Advantageously, the polyamine compounds used in the process according to the invention are chosen from ethylenediamine and polydimethylsiloxanes comprising primary amine groups at the chain end or on side chains.

Preferentially, the amine compounds used in the process according to the invention are chosen from polydimethylsiloxanes comprising primary amine groups at the chain end or on side chains, 3-aminopropyltriethoxysilane (APTES). More preferentially, polydimethylsiloxanes comprising aminopropyl end groups at the chain end, 3-aminopropyltriethoxysilane (APTES), are used.

Advantageously, the amine compound used in the process according to the invention is used in a mole ratio of amine group of the amine compound/phosphonic acid group of the phosphonic polymer ranging from 0.01 to 10, preferably ranging from 0.1 to 5, preferentially ranging from 0.1 to 2 and more preferentially ranging from 0.1 to 1.

On contact with the phosphonic polymer, the polyamine compound reacts with the phosphonic acid functions to form a crosslinked polymer, for example in the following manner:

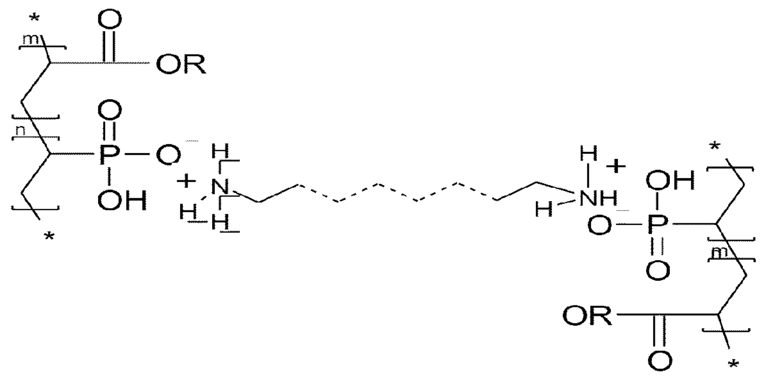

Scheme I

Such a crosslinked polymer is novel and thus also forms the subject of the present invention.

The crosslinked polymer may thus be obtained by reacting said polyamine compound with the vinylphosphonic acid block polymer described previously. Some or all of the anhydride groups react with the NH or NH2 group of the polyamine compound and form a unit bearing an amide group and a carboxylic acid group as described in scheme I.

On contact with the phosphonic polymer, in anhydrous medium, the amino alkoxysilane compound (II) reacts with the phosphonic acid functions to form a unit having the following formula:

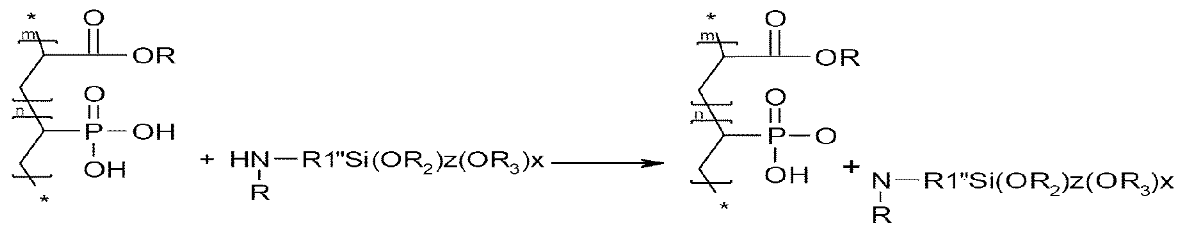

Scheme II

Such a block polymer bearing an amino alkoxysilane group obtained by reacting the phosphonic block polymer with the amino alkoxysilane compound (II) is novel and thus also forms the subject of the present invention. A subject of the invention is also an anhydrous composition comprising such a block polymer bearing an amino alkoxysilane group and a physiologically acceptable medium.
A subject of the invention is thus also a polymer obtained by reacting said phosphonic block polymer with an amine compound as defined previously, the reaction taking place in anhydrous medium when the amine compound is an amino alkoxysilane.

Other particular additional components may be used in the process according to the invention to contribute toward improving the film-forming properties of the polymer according to the invention. Such additional components are especially the salts of divalent or trivalent metal ions, clays and metal oxides described below.

The composition according to the invention may comprise salts of divalent or trivalent metal ions, chosen in particular from salts of ions derived from Al(III), Ca(II), Cu(II), Fe(II), Fe(III), Mg(II), Mn(II), Zn(II), and mixtures thereof.

Ions derived from Ca(II), Mg(II) are preferred.
The salts of these metal ions are well known, with, for example, anions such as gluconate, chloride, sulfate, hydroxide, acetate and stearate. For example, use may be made of the following salts: calcium gluconate, calcium chloride, magnesium chloride, copper chloride, magnesium gluconate, iron sulfate, iron gluconate, aluminium sulfate, sodium stearate. Calcium stearate or zinc acetate is preferably used, and preferentially zinc acetate.
Said salts of divalent or trivalent metal ions may be present in the composition according to the invention in a content ranging from 0.1% to 20% by weight, preferably from 0.1% to 15% by weight, relative to the total weight of the composition.

Alternatively, the salt of divalent or trivalent metal ions may be applied sequentially in the process according to the invention.

The composition according to the invention may comprise a clay.
Clays are products that are already well known per se, which are described, for example, in the publication Mineralogie des argiles [Mineralogy of Clays], S. Caillère, S. Hénin, M. Rautureau, 2nd Edition 1982, Masson, the teaching of which is included herein by way of reference.
Among the clays, examples that may be mentioned include clays of the smectite family, such as laponite and montmorillonite, of the kaolinite family, such as kaolinite, dickite, nacrite, optionally modified clays of the halloysite, dombassite, antigorite, berthierine, pyrophyllite, montmorillonite, beidellite, vermiculite, talc, stevensite, hectorite, bentonite, saponite, chlorite, sepiolite and illite family.
The clay(s) present in the composition of the invention may be natural or synthetic. Natural clay is a sedimentary rock composed to a large extent of specific minerals, silicates generally of aluminium. Kaolin is thus a natural clay.
The clays may also be chemically modified by various compounds, such as acrylic acids, polysaccharides (for example carboxymethylcellulose) or organic cations.
Preferably, in the context of the present invention, use is made of clays that are cosmetically compatible with and acceptable for the skin and/or the scalp.
According to a particular embodiment of the present invention, the clay used is chosen from kaolinite, montmorillonites, saponites, laponites, bentonites, and in particular hectorites, and illites. Use is even more particularly made of mixtures of clays, and natural clays.
Natural clays that may be mentioned include green clays, in particular rich in illite; clays rich in montmorillonite, known as fuller's earth, or such as bentonite or else white clays rich in kaolinite. Bentonites that may be mentioned in particular include those sold under the names Bentone 38 VCG, Bentone Gel CAO V, Bentone 27 V, Bentone Gel MIO V and Bentone Gel ISD V by the company Elementis.
Montmorillonites and smectites are hydrated aluminium and/or magnesium silicates. Examples that may be mentioned include the montmorillonite sold under the name Gel White H by the company Rockwood Additives, and the purified smectite sold under the name Veegum Granules by the company Vanderbilt. Mention may also be made of the montmorillonite sold under the name Kunipia G4 by the company Kunimine and the sepiolite Pangel S9 sold by the company Tolsa.
Examples of kaolinites that may be mentioned include the kaolins sold under the name Coslin C 100 by the company BASF Personal Care Ingredients or Kaolin Supreme by the company Imerys.
Talcs are hydrated magnesium silicates usually comprising aluminium silicate. The crystal structure of talc consists of repeated layers of a sandwich of brucite between layers of silica. Examples that may be mentioned include micronized magnesium silicate of particle size 5 microns sold under the name Micro Ace P3 by the company Nippon Talc or the talcs sold under the names Rose Talc and Talc SG-2000 by the company Nippon Talc, J 68 BC by the company US Cosmetics (Miyoshi), Lyzenac 00 and Luzenac Pharma M by the company Luzenac, and Talc JA-46R by the company Asada Milling.
As saponite, which belongs to the montmorillonite family, mention may be made of synthetic saponite, in particular the product sold by the company Kunimine under the name Sumecton®.
An example of a synthetic laponite that may be mentioned is the laponite XLG sold by the company Rockwood.
The clay may be present in the composition according to the invention in an amount ranging from 0.1% to 50% by weight, especially from 1% to 30% by weight and in particular from 1% to 20% by weight relative to the total weight of the composition.

The metal oxides may be chosen from titanium dioxide, iron oxides, zirconium oxides, zinc oxides, cerium oxides and chromium oxides. Iron oxides or titanium dioxide are preferably used.
The metal oxide may be present in the composition according to the invention in an amount ranging from 0.1% to 50% by weight, especially from 1% to 30% by weight and in particular from 1% to 20% by weight relative to the total weight of the composition.

Advantageously, the process according to the invention is performed under ambient conditions, in particular at an ambient temperature that may range from 15°C to 30°C, preferably ranging from 18°C to 25°C.

The composition used according to the invention is generally suitable for topical application to keratin materials, and thus generally comprises a physiologically acceptable medium, i.e. a medium that is compatible with the keratin materials such as the skin and/or its integuments. It is preferably a cosmetically acceptable medium, i.e. a medium which

has a pleasant colour, odour and feel and which does not cause any unacceptable discomfort (stinging, tautness or redness) liable to discourage the consumer from using this composition.

According to a preferred embodiment of the invention, the composition comprising the phosphonic polymer may contain a hydrocarbon-based oil.

The hydrocarbon-based oil is an oil that is liquid at room temperature (25°C).

The term "hydrocarbon-based oil" means an oil formed essentially from, or even constituted by, carbon and hydrogen atoms, and optionally oxygen and nitrogen atoms, and not containing any silicon or fluorine atoms. It may contain alcohol, ester, ether, carboxylic acid, amine and/or amide groups.

The hydrocarbon-based oil may be volatile or non-volatile.

The hydrocarbon-based oil may be chosen from:

- hydrocarbon-based oils containing from 8 to 14 carbon atoms, and especially:

- branched $C_8$-$C_{14}$ alkanes, for instance $C_8$-$C_{14}$ isoalkanes of petroleum origin (also known as isoparaffins), for instance isododecane (also known as 2,2,4,4,6-pentamethylheptane), isodecane and, for example, the oils sold under the trade name Isopar or Permethyl,

- linear alkanes, for instance n-dodecane (C12) and n-tetradecane (C14) sold by Sasol under the respective references Parafol 12-97 and Parafol 14-97, and also mixtures thereof, the undecane-tridecane mixture, the mixtures of n-undecane (C11) and of n-tridecane (C13) obtained in Examples 1 and 2 of patent application WO 2008/155 059 from the company Cognis, and mixtures thereof,

- short-chain esters (containing from 3 to 8 carbon atoms in total) such as ethyl acetate, methyl acetate, propyl acetate or n-butyl acetate,

- hydrocarbon-based oils of plant origin such as triglycerides consisting of fatty acid esters of glycerol, the fatty acids of which may have chain lengths varying from $C_4$ to $C_{24}$, these chains possibly being linear or branched, and saturated or unsaturated; these oils are especially heptanoic or octanoic acid triglycerides, or alternatively wheatgerm oil, sunflower oil, grapeseed oil, sesame seed oil, corn oil, apricot oil, castor oil, shea oil, avocado oil, olive oil, soybean oil, sweet almond oil, palm oil, rapeseed oil, cottonseed oil, hazelnut oil, macadamia oil, jojoba oil, alfalfa oil, poppy oil, pumpkin oil, marrow oil, blackcurrant oil, evening primrose oil, millet oil, barley oil, quinoa oil, rye oil, safflower oil, candlenut oil, passion-flower oil and musk rose oil; shea butter; or else caprylic/capric acid triglycerides, for instance those sold by the company Stéarineries Dubois or those sold under the names Miglyol 810®, 812® and 818® by the company Dynamit Nobel,

- synthetic ethers having from 10 to 40 carbon atoms;

- linear or branched hydrocarbons of mineral or synthetic origin, such as petroleum jelly, polydecenes, hydrogenated polyisobutene such as Parleam®, squalane and liquid paraffins, and mixtures thereof,

- synthetic esters such as oils of formula $R_1COOR_2$ in which $R_1$ represents a linear or branched fatty acid residue containing from 1 to 40 carbon atoms and $R_2$ represents an, in particular, branched hydrocarbon-based chain containing from 1 to 40 carbon atoms, on condition that $R_1 + R_2 \geq 10$, for instance purcellin oil (cetostearyl octanoate), isopropyl myristate, isopropyl palmitate, $C_{12}$ to $C_{15}$ alkyl benzoates, hexyl laurate, diisopropyl adipate, isononyl isononanoate, 2-ethylhexyl palmitate, isostearyl isostearate, 2-hexyldecyl laurate, 2-octyldecyl palmitate, 2-octyldodecyl myristate, alkyl or polyalkyl heptanoates, octanoates, decanoates or ricinoleates such as propylene glycol dioctanoate; hydroxylated esters such as isostearyl lactate, diisostearyl malate and 2-octyldodecyl lactate; polyol esters and pentaerythritol esters,

- fatty alcohols that are liquid at room temperature, with a branched and/or unsaturated carbon-based chain containing from 12 to 26 carbon atoms, for instance octyldodecanol, isostearyl alcohol, oleyl alcohol, 2-hexyldecanol, 2-butyloctanol and 2-undecylpentadecanol.

Advantageously, the hydrocarbon-based oil is apolar (thus formed solely from carbon and hydrogen atoms).

The hydrocarbon-based oil is preferably chosen from hydrocarbon-based oils containing from 8 to 14 carbon atoms, in particular the apolar oils described previously.

Preferentially, the hydrocarbon-based oil is isododecane.

The composition comprising the polymer may contain, in addition to the hydrocarbon-based oil, a silicone oil. The term

"silicone oil" means an oil comprising at least one silicon atom and especially at least one Si-O group. The silicone oil may be volatile or non-volatile.

The term "volatile oil" means an oil (or non-aqueous medium) that is capable of evaporating on contact with the skin in less than one hour, at room temperature and at atmospheric pressure. The volatile oil is a volatile cosmetic oil, which is liquid at room temperature, especially having a non-zero vapour pressure, at room temperature and at atmospheric pressure, in particular having a vapour pressure ranging from 0.13 Pa to 40 000 Pa ($10^{-3}$ to 300 mmHg), preferably ranging from 1.3 Pa to 13 000 Pa (0.01 to 100 mmHg) and preferentially ranging from 1.3 Pa to 1300 Pa (0.01 to 10 mmHg).

The term "non-volatile oil" means an oil with a vapour pressure of less than 0.13 Pa.

Volatile silicone oils that may be mentioned include volatile linear or cyclic silicone oils, especially those with a viscosity ≤ 8 centistokes (cSt) (8 x $10^{-6}$ $m^2/s$), and especially having from 2 to 10 silicon atoms and in particular from 2 to 7 silicon atoms, these silicones optionally comprising alkyl or alkoxy groups having from 1 to 10 carbon atoms. As volatile silicone oils that may be used in the invention, mention may be made especially of dimethicones with viscosities of 5 and 6 cSt, octamethylcyclotetrasiloxane, decamethylcyclopentasiloxane, dodecamethylcyclohexasiloxane, heptamethylhexyltrisiloxane, heptamethyloctyltrisiloxane, hexamethyldisiloxane, octamethyltrisiloxane, decamethyltetrasiloxane and dodecamethylpentasiloxane, and mixtures thereof.

As non-volatile silicone oils, mention may be made of linear or cyclic non-volatile polydimethylsiloxanes (PDMSs); polydimethylsiloxanes comprising alkyl, alkoxy or phenyl groups, which are pendant or at the end of a silicone chain, these groups containing from 2 to 24 carbon atoms; phenyl silicones, for instance phenyl trimethicones, phenyl dimethicones, phenyltrimethylsiloxydiphenylsiloxanes, diphenyl dimethicones, diphenylmethyldiphenyltrisiloxanes and 2-phenylethyl trimethylsiloxysilicates.

Advantageously, the composition may comprise a hydrocarbon-based oil in a content ranging from 60% to 100% by weight relative to the total weight of the oils present in the composition and from 0 to 40% by weight of silicone oil. According to a preferred embodiment of the invention, the composition contains as oil only a hydrocarbon-based oil.

The composition according to the invention may comprise a cosmetic additive chosen from fragrances, preserving agents, fillers, UV-screening agents, oils, waxes, surfactants, moisturizers, vitamins, ceramides, antioxidants, free-radical scavengers, polymers, thickeners and dyestuffs.

The composition according to the invention may also comprise a dyestuff such as pulverulent dyestuffs, liposoluble dyes or water-soluble dyes. This dyestuff may be present in a content ranging from 0.01% to 30% by weight, relative to the total weight of the composition.

The pulverulent dyestuffs may be chosen from pigments and nacres.

The pigments may be white or coloured, mineral and/or organic, and coated or uncoated. Among the mineral pigments that may be mentioned are titanium dioxide, optionally surface-treated, zirconium, zinc or cerium oxide, and also iron or chromium oxide, manganese violet, ultramarine blue, chromium hydrate and ferric blue. Among the organic pigments that may be mentioned are carbon black, pigments of D&C type and lakes based on cochineal carmine or on barium, strontium, calcium or aluminium.

The nacres may be chosen from white nacreous pigments such as mica coated with titanium or with bismuth oxychloride, coloured nacreous pigments such as titanium mica with iron oxides, titanium mica with in particular ferric blue or chromium oxide, titanium mica with an organic pigment of the abovementioned type, and also nacreous pigments based on bismuth oxychloride.

The liposoluble dyes are, for example, Sudan Red, D&C Red 17, D&C Green 6, β-carotene, soybean oil, Sudan Brown, D&C Yellow 11, D&C Violet 2, D&C Orange 5, quinoline yellow and annatto. The water-soluble dyes are, for example, beetroot juice or methylene blue.

Advantageously, the composition according to the invention is a skincare composition.

The composition according to the invention may be a makeup composition such as a foundation, a lipstick or a liner.

According to one embodiment, the composition according to the invention is a makeup composition and comprises a volatile oil and a non-volatile oil as described previously. In particular, the makeup composition may comprise a hydrocarbon-based volatile oil and a hydrocarbon-based non-volatile oil.

According to one embodiment, the composition used according to the invention is an anhydrous composition. The term "anhydrous composition" means a composition containing less than 2% by weight of water, or even less than 0.5% of water, and is especially free of water. Where appropriate, such small amounts of water may especially be introduced by ingredients of the composition that may contain residual amounts thereof.

In particular, when the amine compound is an amino alkoxysilane (III) as defined previously, the composition containing it and the compositions used in the process are anhydrous compositions. Advantageously, these compositions also contain a C2-C5 monoalcohol such as ethanol or isopropanol, especially in a content ranging from 0.1% to 5% by weight, relative to the total weight of the composition.

The invention will now be described with reference to the following examples, which are given as non-limiting illustrations.

**Example 1:** Isobornyl methacrylate/isobornyl acrylate (35/35)-co-isobutyl acrylate/vinylphosphonic acid (25/5) copolymer

150 g of isododecane were placed in a 1 litre reactor and the solvent was then heated, increasing the temperature from 25°C to 90°C over 1 hour.
105 g of isobornyl methacrylate, 105 g of isobornyl acrylate and 1.8 g of initiator 2,5-bis(2-ethylhexanoylperoxy)-2,5-dimethylhexane (Trigonox® 141 from AkzoNobel) were then added over 1 hour, while maintaining the temperature at 90°C. The mixture was maintained at 90°C for 1 hour 30 minutes. 75 g of isobutyl acrylate, 15 g of vinylphosphonic acid, 1.2 g of 2,5-bis(2-ethylhexanoylperoxy)-2,5-dimethylhexane and 20 g of ethanol were then added over 30 minutes, still at 90°C.
The mixture was maintained at 90°C for 3 hours and was then diluted with 150 g of isododecane, and then concentrated by distillation to remove the unreacted monomers.
A solution containing 50% by weight of the polymer in isododecane was finally obtained.
The polymer obtained has a number-average molecular weight (Mn) of 20 800 and a weight-average molecular weight (Mw) of 3 122 000; with an Ip = 15.

**Examples 2 to 5: Cosmetic evaluation of makeup composition**

The makeup compositions (lip gloss) described below containing the polymer of Example 1 with or without disteardimonium hectorite were prepared, and the composition was then applied onto a skin equivalent support made of elastomer by producing a deposit with a wet thickness of 100 μm, which was left to dry at room temperature (25°C) for 24 hours.
The state of the film obtained was then observed.
The elastomer support was also deformed manually and the state of the film after this deformation was observed to determine its resistance to deformation.
The resistance of the film obtained was evaluated by separately applying 0.5 ml of water, 0.5 ml of olive oil and 0.5 ml of sebum; after 5 minutes of contact, the surface of the film was rubbed with cotton wool and the state of the film was then observed (degraded or undegraded appearance of the film).
The tackiness of the film and its capacity for transferring or not transferring on touching the film with a finger were also evaluated.
The evaluation was made in the following manner:

+++: very efficient evaluated cosmetic property
++: moderately efficient evaluated cosmetic property
+: sparingly efficient evaluated cosmetic property
0: inefficient evaluated cosmetic property

The following results were obtained:

| | Example 2 | Example 3 | Example 4 | Example 5 |
|---|---|---|---|---|
| **Composition** | | | | |
| Polymer of Example 1 | 25 g AM | 25 g AM | 25 g AM | 25 g AM |
| Pigmentary paste containing 40% by weight of pigment in isododecane | 5 g with DC Red 7 | 5 g with DC Red 7 | 5 g with DC Red 7 | 5 g with DC Red 7 |
| Disteardimonium hectorite (Bentone Gel ISD V from Elementis) | - | 10 g | | 10 g |
| 2-Octyldodecanol | - | - | 20 g | 20 |
| Isododecane | 70 g | 60 g | 50 g | 40 g |
| **Evaluation of the film** | | | | |
| Appearance of the film | Homogeneous film | Homogeneous film | Homogeneous film | Homogeneous film |

(continued)

| | | | | |
|---|---|---|---|---|
| **Evaluation of the film** | | | | |
| Resistance to deformation | Yes but with fragmentation of the film | Yes but with fragmentation of the film | Yes without damaging the film | Yes without damaging the film |
| Water resistance | ++ | +++ | ++ | +++ |
| Olive oil resistance | + | +++ | + | ++ |
| Sebum resistance | + | +++ | + | ++ |
| Non-tacky | +++ | +++ | + | ++ |
| Transfer-resistant | +++ | +++ | + | ++ |

The results obtained show that the deposit resulting from the application of polymer 1 alone (Example 2) forms a non-tacky homogeneous film, which does not transfer onto the finger, but which fragments after mechanical stress and which has poor resistance to contact with olive oil and sebum. The deposit resulting from the application of polymer 1 mixed with disteardimonium hectorite (Example 3) forms a homogeneous film, which fragments under mechanical stress, and the resistance of the film to contact with olive oil and sebum is markedly improved.
When the composition comprises 2-octyldodecanol (non-volatile oil), the deposit obtained resulting from the application of polymer 1 alone (Example 4) forms a homogeneous film which is not fragmented after mechanical stress, but which is slightly tacky, not sufficiently transfer-resistant, and sparingly resistant to contact with olive oil and sebum. The deposit resulting from the application of polymer 1 mixed with disteardimonium hectorite (Example 5), for its part, shows an improvement in the tack-free and transfer-resistance properties and in the resistance of the film to contact with olive oil and sebum.
Thus, the addition of disteardimonium hectorite contributes towards improving the cosmetic properties of the film obtained.
The lipstick compositions of Examples 3 and 5 applied to the lips thus make it possible to obtain a non-tacky, transfer-resistant and water-, oil- and sebum-resistant makeup which thus has good persistence.

**Examples 6 and 7: Cosmetic evaluation of makeup composition**

The makeup compositions (lip gloss) described below containing the polymer of Example 1 with or without 3-aminopropyl-terminated polydimethylsiloxane were prepared, and the cosmetic properties were then evaluated according to the protocols described in the preceding Examples 2 to 5.
The following results were obtained:

| | Example 2 | Example 6 | Example 4 | Example 7 |
|---|---|---|---|---|
| **Composition** | | | | |
| Polymer of Example 1 | 25 g AM | 25 g AM | 25 g AM | 25 g AM |
| Pigmentary paste containing 40% by weight of pigment in isododecane | 5 g with DC Red 7 | 5 g with DC Red 7 | 5 g with DC Red 7 | 5 g with DC Red 7 |
| 3-Aminopropyl-terminated polydimethylsiloxane (Mn 2500; reference 481688 from Sigma) | - | 2.5 g | - | 2.5 g |
| 2-Octyldodecanol | - | - | 20 g | 20 |
| Isododecane | 70 g | 67.5 g | 50 g | 47.5 g |
| **Evaluation of the film** | | | | |
| Appearance of the film | Homogeneous film | Homogeneous film | Homogeneous film | Homogeneous film |

(continued)

| | | | | |
|---|---|---|---|---|
| **Evaluation of the film** | | | | |
| Resistance to deformation | Yes but with fragmentation of the film | Yes but with fragmentation of the film | Yes without damaging the film | Yes without damaging the film |
| Olive oil resistance | + | +++ | + | ++ |
| Sebum resistance | + | +++ | + | ++ |
| Non-tacky | +++ | +++ | + | ++ |
| Transfer-resistant | +++ | +++ | + | ++ |

The deposit resulting from the application of polymer 1 mixed with 3-aminopropyl-terminated polydimethylsiloxane (Example 6) forms a homogeneous film, which fragments under mechanical stress, and the resistance of the film to contact with olive oil and sebum is markedly improved (by comparison with Example 2).

When the composition comprises 2-octyldodecanol (non-volatile oil), the deposit resulting from the application of polymer 1 mixed with 3-aminopropyl-terminated polydimethylsiloxane (Example 7) shows an improvement in the tack-free and transfer-resistance properties and in the resistance of the film to contact with olive oil and sebum. Thus the addition of the 3-aminopropyl-terminated polydimethylsiloxane contributes towards improving the cosmetic properties of the film obtained (by comparison with Example 4).

The lipstick compositions of Examples 6 and 7 applied to the lips thus make it possible to obtain a non-tacky, transfer-resistant and water-, oil- and sebum-resistant makeup which thus has good persistence.

**Examples 8 and 9: Cosmetic evaluation of makeup composition**

The makeup compositions described below containing the polymer of Example 1 with or without APTES were prepared, and the cosmetic properties were then evaluated according to the protocols described in the preceding Examples 2 to 5.

The following results were obtained:

| | Example 2 | Example 8 | Example 4 | Example 9 |
|---|---|---|---|---|
| **Composition** | | | | |
| Polymer of Example 1 | 25 g AM | 25 g AM | 25 g AM | 25 g AM |
| Pigmentary paste containing 40% by weight of pigment in isododecane | 5 g with DC Red 7 | 5 g with DC Red 7 | 5 g with DC Red 7 | 5 g with DC Red 7 |
| APTES | - | 2.5 g | - | 2.5 g |
| Isopropanol | | 1 g | | 1 g |
| 2-Octyldodecanol | - | - | 20 g | 20 g |
| Isododecane | qs 100 g | qs 100 g | qs 100 g | qs 100 g |
| **Evaluation of the film** | | | | |
| Appearance of the film | Homogeneous film | Homogeneous film | Homogeneous film | Homogeneous film |
| Resistance to deformation | Yes but with fragmentation of the film | Yes but with fragmentation of the film | Yes without damaging the film | Yes without damaging the film |
| Water resistance | ++ | +++ | ++ | +++ |
| Olive oil resistance | + | +++ | + | ++ |
| Sebum resistance | + | +++ | + | ++ |

(continued)

| | | | | |
|---|---|---|---|---|
| **Evaluation of the film** | | | | |
| Non-tacky | +++ | +++ | + | ++ |
| Transfer-resistant | +++ | +++ | + | ++ |

The deposit resulting from the application of polymer 1 mixed with APTES (Example 8) forms a homogeneous film, which fragments under mechanical stress, and the resistance of the film to contact with olive oil and sebum is markedly improved (by comparison with Example 2).
When the composition comprises 2-octyldodecanol (non-volatile oil), the deposit resulting from the application of polymer 1 mixed with APTES (Example 9) shows an improvement in the tack-free and transfer-resistance properties and in the resistance of the film to contact with water, olive oil and sebum.
Thus the addition of the APTES contributes towards improving the cosmetic properties of the film obtained (by comparison with Example 4).
The lipstick compositions of Examples 8 and 9 applied to the lips thus make it possible to obtain a non-tacky, transfer-resistant and water-, oil- and sebum-resistant makeup which thus has good persistence.

**Examples 10 and 11: Cosmetic evaluation of makeup composition**

The makeup compositions (lip gloss) described below containing the polymer of Example 1 with or without calcium stearate or zinc acetate were prepared, and the cosmetic properties were then evaluated according to the protocols described in the preceding Examples 2 to 5.
The following results were obtained:

| | Example 2 | Example 10 | Example 11 |
|---|---|---|---|
| **Composition** | | | |
| Polymer of Example 1 | 25 g AM | 25 g AM | 25 g AM |
| Pigmentary paste containing 40% by weight of pigment in isododecane | 5 g with DC Red 7 | 5 g with DC Red 7 | 5 g with DC Red 7 |
| Calcium stearate | - | 3.75 g | - |
| Zinc acetate | - | - | 3.75 g |
| Isododecane | qs 100 g | qs 100 g | qs 100 g |
| **Evaluation of the film** | | | |
| Appearance of the film | Homogeneous film | Homogeneous film | Homogeneous film |
| Resistance to deformation | Yes but with fragmentation of the film | Yes but with fragmentation of the film | Yes but with fragmentation of the film |
| Olive oil resistance | + | +++ | +++ |
| Sebum resistance | + | +++ | +++ |
| Non-tacky | +++ | +++ | +++ |
| Transfer-resistant | +++ | +++ | +++ |

The deposits resulting from the application of polymer 1 mixed with calcium stearate (Example 10) or zinc acetate (Example 11) form a homogeneous film, which fragments under mechanical stress, and the resistance of the film to contact with olive oil and sebum is markedly improved (by comparison with Example 2).
Thus the addition of calcium stearate (Example 10) or zinc acetate (Example 11) contributes towards improving the cosmetic properties of the film obtained (by comparison with Example 2).
The lipstick compositions of Examples 10 and 11 applied to the lips thus make it possible to obtain a non-tacky, transfer-resistant and water-, oil- and sebum-resistant makeup which thus has good persistence.

**Claims**

1. Block polymer comprising:

   at least one first block with a glass transition temperature (Tg) of greater than or equal to 40°C and obtained from at least one (meth)acrylate monomer of formula $CH_2 = C(R_1)\text{-}COOR_2$ in which $R_1$ represents H or a methyl radical and $R_2$ represents a $C_4$ to $C_{12}$ cycloalkyl group; and
   at least one second block with a glass transition temperature (Tg) of less than or equal to 20°C and is obtained from at least one vinylphosphonic acid monomer of formula (I) and from at least one (meth)acrylate monomer of formula $CH_2 = C(R_1)\text{-}COOR_3$ in which $R_1$ represents H or a methyl radical and $R_3$ represents either a linear or branched $C_1$ to $C_6$ unsubstituted alkyl group, with the exception of a tert-butyl group or a methoxyethyl group; said vinylphosphonic acid monomer of formula (I) being:

   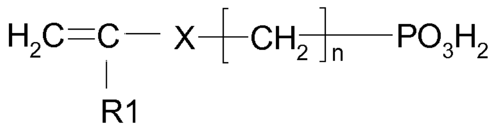

   (I)

   in which:

   R1 denotes H or-CH3;
   X denotes a covalent bond and n denotes an integer ranging from 0 to 14;
   or X denotes a -COO- group and n denotes an integer ranging from 2 to 6.

2. Polymer according to the preceding claim, **characterized in that** the first block is obtained from at least one acrylate monomer of formula $CH_2 = CH\text{-}COOR_2$ in which $R_2$ represents a $C_4$ to $C_{12}$ cycloalkyl group, and from at least one methacrylate monomer of formula $CH_2 = C(CH_3)\text{-}COOR'_2$ in which $R'_2$ represents a $C_4$ to $C_{12}$ cycloalkyl group; and optionally an additional monomer chosen from linear or branched C8-C22 alkyl (meth)acrylates.

3. Polymer according to the preceding claim, **characterized in that** for the first block, said acrylate monomer and said methacrylate monomer are in acrylate/methacrylate mass proportions of between 30/70 and 70/30, preferably between 40/60 and 60/40, especially between 45/55 and 55/45.

4. Polymer according to either of Claims 2 and 3, **characterized in that** the first block is obtained by polymerization of isobornyl methacrylate and isobornyl acrylate.

5. Polymer according to any one of the preceding claims, **characterized in that** the proportion of the first block ranges from 60% to 80% and better still from 65% to 75% by weight of the polymer.

6. Polymer according to any one of the preceding claims, **characterized in that**, for the monomer (I):
   X denotes a covalent bond and n is an integer ranging from 0 to 6 or X denotes a -COO-group and n is an integer ranging from 2 to 4.

7. Polymer according to one of the preceding claims, **characterized in that**, for monomer (I), R1 = H and X denotes a covalent bond and n is an integer ranging from 0 to 4.

8. Polymer according to one of the preceding claims, **characterized in that** monomer (I) is chosen from:

   vinylphosphonic acid;
   3-butenylphosphonic acid;
   4-pentenylphosphonic acid;
   10-undecenylphosphonic acid;
   11-dodecenylphosphonic acid;
   2-phosphonoethyl ester of 2-methyl-2-propenoic acid;
   2-phosphonoethyl ester of 2-propenoic acid;
   and preferably vinylphosphonic acid.

**9.** Polymer according to one of the preceding claims, **characterized in that** the second block comprises a monomer chosen from isobutyl acrylate, ethyl acrylate, n-butyl acrylate and methoxyethyl acrylate, or mixtures thereof, and preferably isobutyl acrylate.

**10.** Polymer according to any one of the preceding claims, **characterized in that**, for the second block, the vinylphosphonic acid monomer (I) and said (meth)acrylate monomer are in (meth)acrylate/vinylphosphonic acid (I) mass proportions ranging from 1 to 10, preferentially ranging from 2 to 9, especially ranging from 3 to 8, or alternatively ranging from 4 to 7.

**11.** Polymer according to one of the preceding claims, **characterized in that** the proportion of the second block ranges from 20% to 40% and better still from 25% to 35% by weight of the polymer.

**12.** Polymer according to one of the preceding claims, **characterized in that** it comprises an intermediate segment comprising at least one constituent monomer of the first block and at least one constituent monomer of the second block, and is especially a statistical polymer.

**13.** Polymer according to one of the preceding claims, **characterized in that** it has a polydispersity index of greater than 2, preferably ranging from 3 to 20.

**14.** Composition comprising, in a physiologically acceptable medium, a block polymer according to one of Claims 1 to 13.

**15.** Composition according to the preceding claim, **characterized in that** the block polymer is present in a content ranging from 0.1% to 40% by weight, relative to the total weight of the composition derived from the extemporaneous mixture, preferably from 0.5% to 35% by weight, preferentially ranging from 1% to 30% by weight, and more preferentially ranging from 10% to 30% by weight.

**16.** Cosmetic process for treating keratin materials, comprising the topical application to the keratin materials of a composition according to Claim 14 or 15.

**17.** Process according to the preceding claim, **characterized in that** a composition obtained by mixing the composition according to either of Claims 14 and 15 and an additional component chosen from:

(i) an amine compound chosen from polyamine compounds bearing several primary amine groups and/or secondary amine groups and amino alkoxysilanes,
(ii) salts of divalent or trivalent metal ions,
(iii) clays,
(iv) metal oxides,

or a composition containing same and comprising a physiologically acceptable medium, is applied topically to keratin materials, the composition(s) used being anhydrous when the additional component is an amino alkoxysilane.

**18.** Process according to the preceding claim, **characterized in that** the polyamine compound comprises from 2 to 20 carbon atoms.

**19.** Process according to either of Claims 17 and 18, **characterized in that** the polyamine compound is chosen from N-methyl-1,3-diaminopropane, N-propyl-1,3-diaminopropane, N-isopropyl-1,3-diaminopropane, N-cyclohexyl-1,3-diaminopropane, 2-(3-aminopropylamino)ethanol, 3-(2-aminoethyl)aminopropylamine, bis(3-aminopropyl)amine, methylbis(3-aminopropyl)amine, N-(3-aminopropyl)-1,4-diaminobutane, N,N-dimethyldipropylenetriamine, 1,2-bis(3-aminopropylamino)ethane, N,N'-bis(3-aminopropyl)-1,3-propanediamine, ethylenediamine, 1,3-propylenediamine, 1,4-butylenediamine, lysine, cystamine, xylenediamine, tris(2-aminoethyl)amine and spermidine.

**20.** Process according to Claim 17, **characterized in that** the amino alkoxysilane is of formula (III):

$$R'_1Si(OR'_2)_z(R'_3)_x \qquad (III)$$

in which:

• $R'_1$ is a linear or branched, saturated or unsaturated, cyclic or acyclic $C_1$-$C_6$ hydrocarbon-based chain sub-

stituted with a group chosen from the following groups:

- amine $NH_2$ or NHR with R = $C_1$-$C_4$ alkyl,
- an aryl or aryloxy group substituted with an amino group or with a $C_1$-$C_4$ aminoalkyl group,

R'$_1$ possibly being interrupted in its chain with a heteroatom (O, S, NH) or a carbonyl group (CO), R'1 being linked to the silicon atom directly via a carbon atom,

• R'$_2$ and R'$_3$, which may be identical or different, represent a linear or branched alkyl group comprising from 1 to 6 carbon atoms,
• z denotes an integer ranging from 1 to 3, and
• x denotes an integer ranging from 0 to 2,

with z + x = 3;
and is preferably 3-aminopropyltriethoxysilane.

**21.** Process according to Claim 17, **characterized in that** the polyamine compound is chosen from amine-based polymers, especially having a weight-average molecular weight ranging from 500 to 1 000 000, preferably ranging from 500 to 500 000, and preferentially ranging from 500 to 100 000.

**22.** Process according to the preceding claim, **characterized in that** the polyamine compound is an amine-based polymer chosen from poly(($C_2$-$C_5$)alkyleneimines), and in particular polyethyleneimines and polypropyleneimines, especially poly(ethyleneimine)s; poly(allylamine); polyvinylamines and copolymers thereof, in particular with vinylamides; vinylamine/vinylformamide copolymers; polyamino acids bearing NH2 groups, such as polylysine; aminodextran; amino polyvinyl alcohol, acrylamidopropylamine-based copolymers; chitosans;
polydimethylsiloxanes comprising primary amine groups at the chain end or on side chains, for example aminopropyl side or end groups, for instance those of formula (A) or (B) or (C):

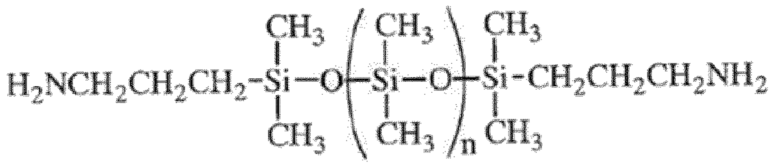

(A)

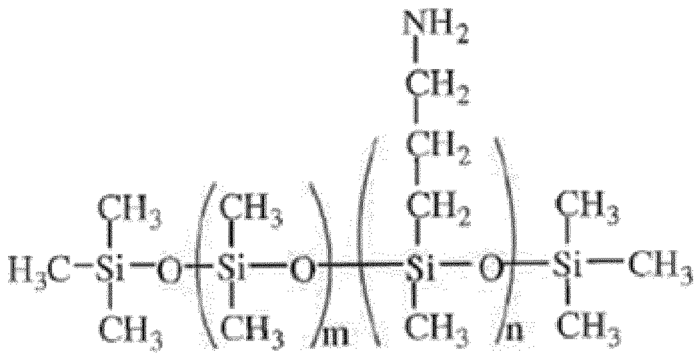

(B)

H2NCH2CH2CH2-Si(CH3)2-O-[Si(CH3)2-O]n-Si(CH3)2C4H9 (C)

with:

in formula (A): the value of n is such that the weight-average molecular weight of the silicone is between 500 and 55 000;
in formula (B), the values of n and m are such that the weight-average molecular weight of the silicone is between 1000 and 55 000;
in formula (C), the value of n is such that the weight-average molecular weight of the silicone is between 500

and 3000;

the amodimethicones of formula (D):

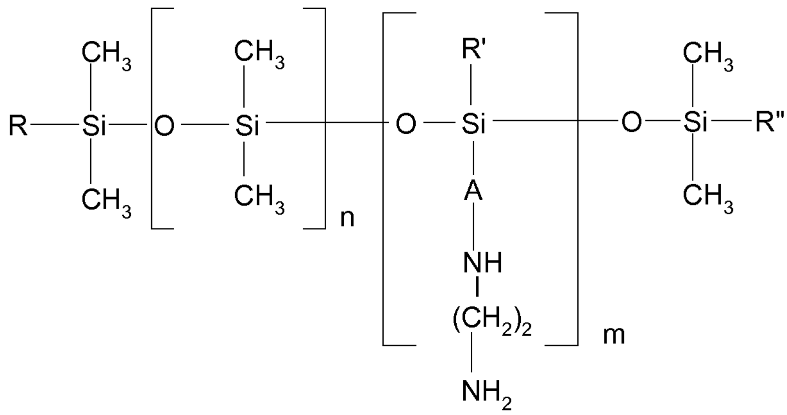

(D)

in which R, R' and R", which may be identical or different, each represent a $C_1$-$C_4$ alkyl or hydroxyl group, A represents a $C_3$ alkylene group and m and n are such that the weight-average molecular mass of the compound is between 5000 and 500 000 approximately;
the amodimethicones of formula (K):

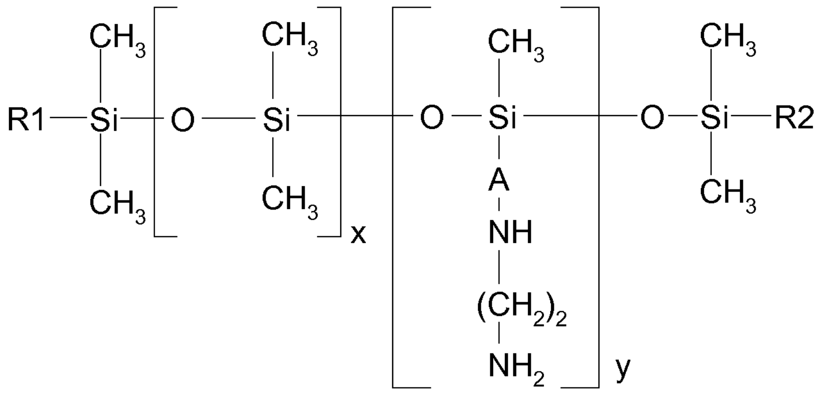

(K)

in which:

- R1 and R2, which may be identical or different, preferably identical, represent a linear or branched, saturated or unsaturated alkyl group comprising from 6 to 30 carbon atoms, preferably from 8 to 24 carbon atoms and preferentially from 12 to 20 carbon atoms,
- A represents a linear or branched alkylene radical group containing from 2 to 8 carbon atoms,
- x and y are numbers ranging from 1 to 5000; preferably, x ranges from 10 to 2000 and especially from 100 to 1000; preferably, y ranges from 1 to 100.

Preferentially, for the amodimethicone of formula (K):

- x ranges from 10 to 2000 and especially from 100 to 1000;
- y ranges from 1 to 100;
- A comprises from 3 to 6 carbon atoms, and in particular 4 carbon atoms; preferably, A is branched; preferentially, A is chosen from the divalent radicals: $-CH_2CH_2CH_2-$ and
- $CH_2CH(CH_3)CH_2-$; and
- R1 and R2, which may be identical or different, represent a saturated linear radical comprising from 6 to 30 carbon atoms, preferably from 8 to 24 carbon atoms and especially from 12 to 20 carbon atoms, for instance a dodecyl, tetradecyl, pentadecyl, hexadecyl, heptadecyl, octadecyl, nonadecyl or eicosyl group. Advantageously, R1 and R2 represent a mixture of hexadecyl (cetyl) and octadecyl (stearyl) radicals (mixture also known as

cetearyl);

preferably, the amodimethicone of formula (K) is bis-cetearyl amodimethicone; polyetherdiamines and especially polyethylene glycol and/or polypropylene glycol α,ω-diamines; polytetrahydrofuran (or polytetramethylene glycol) α,ω-diamines and polybutadiene α,ω-diamines;
polyamidoamine dendrimers bearing amine end functions;
poly(meth)acrylates or poly(meth)acrylamides bearing primary or secondary amine side functions, such as poly(3-aminopropyl)methacrylamide or poly(2-aminoethyl) methacrylate;
and preferably polydimethylsiloxanes comprising primary amine groups at the chain end or on side chains;
preferentially, polydimethylsiloxanes comprising aminopropyl end groups at the chain end.

**23.** Process according to Claim 17, **characterized in that** the additional component is an amine compound chosen from polydimethylsiloxanes comprising primary amine groups at the chain end or on side chains and 3-aminopropyltriethoxysilane; and preferably from polydimethylsiloxanes comprising aminopropyl end groups at the chain end and 3-aminopropyltriethoxysilane.

**24.** Process according to any one of Claims 17 to 23, **characterized in that** the amine compound is used in a mole ratio of amine group of the amine compound/phosphonic acid of the polymer ranging from 0.01 to 10, preferably ranging from 0.1 to 5, preferentially ranging from 0.1 to 2 and more preferentially ranging from 0.1 to 1.

**25.** Process according to either of Claims 17 and 20, **characterized in that** when the composition(s) used contains an amino alkoxysilane, it comprises a C2-C5 monoalcohol, preferably ethanol or isopropanol, especially in a content ranging from 0.1% to 5% by weight, relative to the total weight of the composition.

**26.** Process according to Claim 17, **characterized in that** the additional component is a clay chosen from clays of the smectite family, such as laponite and montmorillonite, of the kaolinite family, such as kaolinite, dickite, nacrite, optionally modified clays of the halloysite, dombassite, antigorite, berthierine, pyrophyllite, montmorillonite, beidellite, vermiculite, talc, stevensite, hectorite, bentonite, saponite, chlorite, sepiolite and illite family; and preferably chosen from kaolinite, smectites such as laponite and montmorillonite, bentonite and saponite.

**27.** Process according to Claim 17, **characterized in that** the additional component is a salt of divalent or trivalent metal ions chosen from salts of ions derived from Al(III), Ca(II), Cu(II), Fe(II), Fe(III), Mg(II), Mn(II), Zn(II) and mixtures thereof; preferably chosen from calcium stearate and zinc acetate.

**28.** Process according to Claim 17, **characterized in that** the additional component is a metal oxide chosen from titanium dioxide, iron oxides, zirconium oxides, zinc oxides, cerium oxides and chromium oxides; preferably chosen from iron oxides and titanium dioxide.

**29.** Process according to any one of Claims 17 to 28, **characterized in that** the mixing of the composition comprising the phosphonic polymer and the additional component, or of the composition containing same, is performed in a time of between 1 minute and 24 hours before application thereof to keratin materials, and preferably between 5 and 30 minutes.

**30.** Process according to any one of Claims 17 to 29, **characterized in that** the composition applied to the keratin materials comprises an oil.

**31.** Composition obtained by mixing a composition according to Claim 14 or 15 and an additional component as defined in one of Claims 17 to 23 and 26 to 28, or a composition containing same and comprising a physiologically acceptable medium.

**32.** Kit comprising a first composition according to Claim 14 or 15 and a second composition comprising an additional compound as defined in one of Claims 17 to 23 and 26 to 28 and comprising a physiologically acceptable medium, the first and second compositions each being packaged in a separate packaging assembly.

**33.** Polymer obtained by reacting a block polymer according to one of Claims 1 to 13 with an amine compound as defined according to one of Claims 17 to 24, the reaction taking place in anhydrous medium when the amine compound is an amino alkoxysilane.

**Patentansprüche**

1. Blockpolymer, umfassend:

   mindestens einen ersten Block, der eine Glasübergangstemperatur (Tg) größer oder gleich 40 °C aufweist und aus mindestens einem (Meth)acrylat-Monomer der Formel $CH_2=C(R_1)-COOR_2$, worin $R_1$ für H oder einen Methylrest steht und $R_2$ für eine $C_4$- bis $C_{12}$-Cycloalkylgruppe steht, erhalten wurde; und
   mindestens einen zweiten Block, der eine Glasübergangstemperatur (Tg) kleiner oder gleich 20 °C aufweist und aus mindestens einem Vinylphosphonsäure-Monomer der Formel (I) und aus mindestens einem (Meth) acrylat-Monomer der Formel $CH_2=C\ (R_1)-COOR_3$, worin $R_1$ für H oder einen Methylrest steht und $R_3$ für eine lineare oder verzweigte unsubstituierte $C_1$- bis $C_6$-Alkylgruppe mit Ausnahme einer tert-Butylgruppe oder einer Methoxyethylgruppe steht, erhalten wurde;
   wobei es sich bei dem Vinylphosphonsäure-Monomer der Formel (I) um

   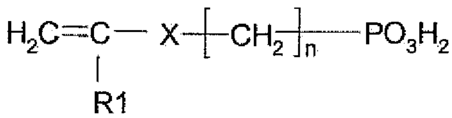

   (I)

   handelt, worin:

   R1 für H oder $-CH_3$ steht;
   X für eine kovalente Bindung steht und n für eine ganze Zahl im Bereich von 0 bis 14 steht oder X für eine -COO-Gruppe steht und n für eine ganze Zahl im Bereich von 2 bis 6 steht.

2. Polymer nach dem vorhergehenden Anspruch, **dadurch gekennzeichnet, dass** der erste Block aus mindestens einem Acrylat-Monomer der Formel $CH_2=CH-COOR_2$, worin $R_2$ für eine $C_4$- bis $C_{12}$-Cycloalkylgruppe steht, und aus mindestens einem Methacrylat-Monomer der Formel $CH_2=C(CH_3)=CH-COOR'_2$, worin $R'_2$ für eine $C_4$- bis $C_{12}$-Cycloalkylgruppe steht, und gegebenenfalls einem zusätzlichen Monomer, das aus linearen oder verzweigten $C_8$-$C_{22}$-Alkyl(meth)acrylaten ausgewählt ist, erhalten wurde.

3. Polymer nach dem vorhergehenden Anspruch, **dadurch gekennzeichnet, dass** für den ersten Block das Acrylat-Monomer und das Methacrylat-Monomer in Acrylat/Methacrylat-Massenanteilen zwischen 30/70 und 70/30, vorzugsweise zwischen 40/60 und 60/40, insbesondere zwischen 45/55 und 55/45, vorliegen.

4. Polymer nach einem der Ansprüche 2 und 3, **dadurch gekennzeichnet, dass** der erste Block durch Polymerisation von Isobornylmethacrylat und Isobornylacrylat erhalten wurde.

5. Polymer nach einem der vorhergehenden Ansprüche, **dadurch gekennzeichnet, dass** der Anteil des ersten Blocks im Bereich von 60 bis 80 Gew. -% und noch besser von 65 bis 75 Gew.-% des Polymers liegt.

6. Polymer nach einem der vorhergehenden Ansprüche, **dadurch gekennzeichnet, dass** für das Monomer (I):
   X für eine kovalente Bindung steht und n für eine ganze Zahl im Bereich von 0 bis 6 steht oder X für eine -COO-Gruppe steht und n für eine ganze Zahl im Bereich von 2 bis 4 steht.

7. Polymer nach einem der vorhergehenden Ansprüche, **dadurch gekennzeichnet, dass** für Monomer (I) R1 = H und X für eine kovalente Bindung steht und n für eine ganze Zahl im Bereich von 0 bis 4 steht.

8. Polymer nach einem der vorhergehenden Ansprüche, **dadurch gekennzeichnet, dass** Monomer (I) aus
   Vinylphosphonsäure;
   3-Butenylphosphonsäure;
   4-Pentenylphosphonsäure;
   10-Undecenylphosphonsäure;
   11-Dodecenylphosphonsäure;
   2-Phosphonoethylester von 2-Methyl-2-propensäure;
   2-Phosphonbethylester von 2-Propensäure;
   und vorzugsweise Vinylphosphonsäure ausgewählt ist.

**9.** Polymer nach einem der vorhergehenden Ansprüche, **dadurch gekennzeichnet, dass** der zweite Block ein Monomer umfasst, das aus Isobutylacrylat, Ethylacrylat, n-Butylacrylat und Methoxyethylacrylat oder Mischungen davon und vorzugsweise Isobutylacrylat ausgewählt ist.

**10.** Polymer nach einem der vorhergehenden Ansprüche, **dadurch gekennzeichnet, dass** für den zweiten Block das Vinylphosphonsäure-Monomer (I) und das (Meth)-acrylat-Monomer in Massenanteilen von (Meth)-acrylat zu Vinylphosphonsäure (I) im Bereich von 1 bis 10, vorzugsweise im Bereich von 2 bis 9, insbesondere im Bereich von 3 bis 8 oder alternativ dazu im Bereich von 4 bis 7 vorliegen.

**11.** Polymer nach einem der vorhergehenden Ansprüche, **dadurch gekennzeichnet, dass** der Anteil des zweiten Blocks im Bereich von 20 bis 40 Gew.-% und noch besser von 25 bis 35 Gew.-% des Polymers liegt.

**12.** Polymer nach einem der vorhergehenden Ansprüche, **dadurch gekennzeichnet, dass** es ein Zwischensegment umfasst, das mindestens ein Aufbaumonomer des ersten Blocks und mindestens ein Aufbaumonomer des zweiten Blocks umfasst und insbesondere ein statistisches Polymer ist.

**13.** Polymer nach einem der vorhergehenden Ansprüche, **dadurch gekennzeichnet, dass** es einen Polydispersitätsindex von mehr als 2, vorzugsweise im Bereich von 3 bis 20, aufweist.

**14.** Zusammensetzung, die ein Blockpolymer nach einem der Ansprüche 1 bis 13 in einem physiologisch unbedenklichen Medium umfasst.

**15.** Zusammensetzung nach dem vorhergehenden Anspruch, **dadurch gekennzeichnet, dass** das Blockpolymer in einem Gehalt im Bereich von 0,1 bis 40 Gew.-%, bezogen auf das Gesamtgewicht der von der extemporalen Mischung abgeleiteten Zusammensetzung, vorzugsweise von 0,5 bis 35 Gew.-%, bevorzugt im Bereich von 1 bis 30 Gew.-% und weiter bevorzugt im Bereich von 10 bis 30 Gew.-% vorliegt.

**16.** Kosmetisches Verfahren zur Behandlung von Keratinmaterialien, umfassend das topische Aufbringen einer Zusammensetzung nach Anspruch 14 oder 15 auf die Keratinmaterialien.

**17.** Verfahren nach dem vorhergehenden Anspruch, **dadurch gekennzeichnet, dass** eine durch Mischen der Zusammensetzung nach Anspruch 14 oder 15 und einer zusätzlichen Komponente, die aus

(i) einer Aminverbindung, die aus Polyaminverbindungen mit mehreren primären Amingruppen und/oder sekundären Amingruppen und Aminoalkoxysilanen ausgewählt ist,
(ii) Salzen von zweiwertigen oder dreiwertigen Metallionen,
(iii) Tonen,
(iv) Metalloxiden

ausgewählt ist, erhaltene Zusammensetzung oder eine Zusammensetzung, die dieselbe enthält und ein physiologisch unbedenkliches Medium umfasst, topisch auf Keratinmaterialien aufbringt, wobei die verwendete Zusammensetzung bzw. die verwendeten Zusammensetzungen wasserfrei ist bzw. sind, wenn es sich bei der zusätzlichen Komponente um ein Aminoalkoxysilan handelt.

**18.** Verfahren nach dem vorhergehenden Anspruch, **dadurch gekennzeichnet, dass** die Polyaminverbindung 2 bis 20 Kohlenstoffatome aufweist.

**19.** Verfahren nach Anspruch 17 oder 18, **dadurch gekennzeichnet, dass** die Polyaminverbindung aus N-Methyl-1,3-diaminopropan, N-Propyl-1,3-diaminopropan, N-Isopropyl-1,3-diaminopropan, N-Cyclohexyl-1,3-diaminopropan, 2-(3-Aminopropylamino)-ethanol, 3-(2-Aminoethyl)aminopropylamin, Bis(3-aminopropyl)amin, Methylbis(3-aminopropyl)amin, N-(3-Aminopropyl)-1,4-diaminobutan, N,N-Dimethyldipropylentriamin, 1,2-Bis(3-aminopropylamino)-ethan, N,N'-Bis(3-aminopropyl)-1,3-propandiamin, Ethylendiamin, 1,3-Propylendiamin, 1,4-Butylendiamin, Lysin, Cystamin, Xyloldiamin, Tris(2-aminoethyl)amin und Spermidin ausgewählt wird.

**20.** Verfahren nach Anspruch 17, **dadurch gekennzeichnet, dass** das Aminoalkoxysilan die Formel (III) aufweist:

$$R'_1Si(OR'_2)_z(R'_3)_x \qquad (III)$$

worin:

- $R'_1$ für eine lineare oder verzweigte, gesättigte oder ungesättigte, cyclische oder acyclische $C_1$-$C_6$-Kohlenwasserstoffkette steht, die durch eine Gruppe substituiert ist, die aus den folgenden Gruppen ausgewählt ist:

  - Amin $NH_2$ oder NHR mit R = $C_1$-$C_4$-Alkyl,
  - einer Aryl- oder Aryloxygruppe, die durch eine Aminogruppe oder durch eine $C_1$-$C_4$-Aminoalkylgruppe substituiert ist,

  wobei $R'_1$ in seiner Kette durch ein Heteroatom (O, S, NH) oder eine Carbonylgruppe (CO) unterbrochen sein kann, wobei $R'_1$ über ein Kohlenstoffatom direkt an das Siliciumatom gebunden ist,

- $R'_2$ und $R'_3$ gleich oder verschieden sind und für eine lineare oder verzweigte Alkylgruppe mit 1 bis 6 Kohlenstoffatomen stehen,
- z für eine ganze Zahl im Bereich von 1 bis 3 steht und
- x für eine ganze Zahl im Bereich von 0 bis 2 steht,

wobei z + x = 3;
und vorzugsweise 3-Aminopropyltriethoxysilan ist.

**21.** Verfahren nach Anspruch 17, **dadurch gekennzeichnet, dass** die Polyaminverbindung aus aminbasierten Polymeren, insbesondere mit einem gewichtsmittleren Molekulargewicht im Bereich von 500 bis 1.000.000, vorzugsweise im Bereich von 500 bis 500.000 und bevorzugt im Bereich von 500 bis 100.000 ausgewählt ist.

**22.** Verfahren nach dem vorhergehenden Anspruch, **dadurch gekennzeichnet, dass** es sich bei der Polyaminverbindung um ein aminbasiertes Polymer handelt, das aus Poly(($C_2$-$C_5$)alkyleniminen) und insbesondere Polyethyleniminen und Polypropyleniminen, insbesondere Poly(ethylenimin)en; Poly-(allylamin) ; Polyvinylaminen und Copolymeren davon, insbesondere mit Vinylamiden; Vinylamin/Vinylformamid-Copolymeren; Polyaminosäuren mit $NH_2$-Gruppen, wie Polylysin; Aminodextran; Aminopolyvinylalkohol, Copolymeren auf Basis von Acrylamidopropylamin; Chitosanen; Polydimethylsiloxanen mit primären Amingruppen am Kettenende oder an Seitenketten, beispielsweise Aminopropyl-Seitengruppen oder -Endgruppen, beispielsweise denjenigen der Formel (A) oder (B) oder (C):

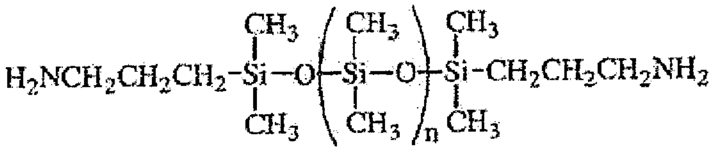

(A)

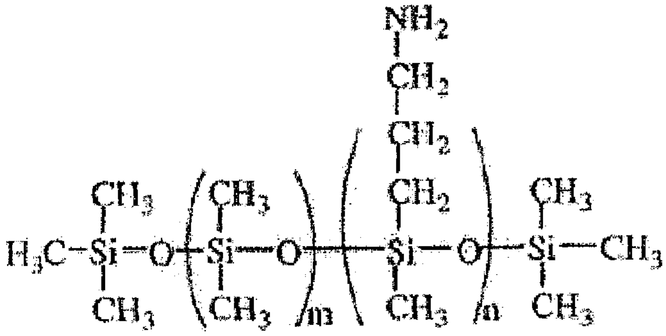

(B)

$$H_2NCH_2CH_2CH_2\text{-}Si(CH_3)_2\text{-}O\text{-}[Si(CH_3)_2\text{-}O]_n\text{-}Si(CH_3)_2C_4H_9 \qquad (C)$$

wobei:

in Formel (A) der Wert von n so beschaffen ist, dass das gewichtsmittlere Molekulargewicht des Silikons zwischen

500 und 55.000 liegt;
in Formel (B) die Werte von n und m so beschaffen sind, dass das gewichtsmittlere Molekulargewicht des Silikons zwischen 1000 und 55.000 liegt;
in Formel (C) der Wert von n so beschaffen ist, dass das gewichtsmittlere Molekulargewicht des Silikons zwischen 500 und 3000 liegt;

den Amodimethiconen der Formel (D):

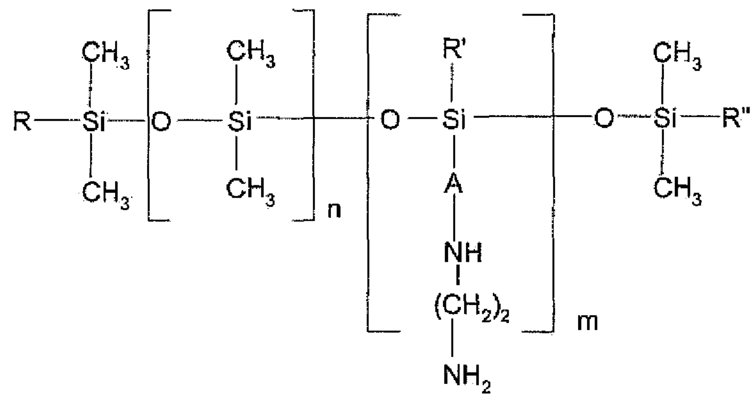

(D)

worin R, R' und R", die gleich oder verschieden sein können, jeweils für eine $C_1$-$C_4$-Alkyl- oder Hydroxylgruppe stehen, A für eine $C_3$-Alkylengruppe steht und m und n so beschaffen sind, dass das gewichtsmittlere Molekulargewicht der Verbindung zwischen ungefähr 5000 und 500.000 liegt;
den Amodimethiconen der Formel (K):

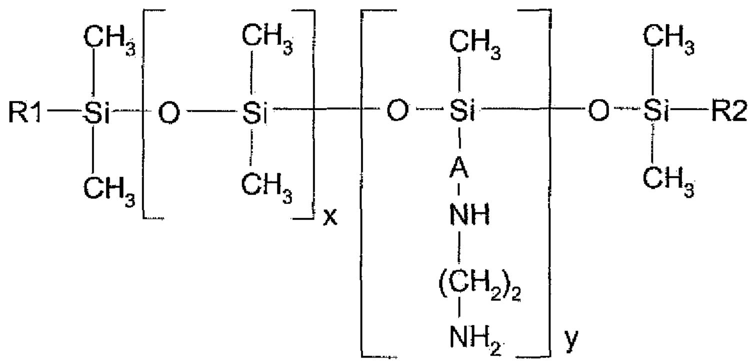

(K)

worin:

- R1 und R2, die gleich oder verschieden, vorzugsweise identisch, sein können, für eine lineare oder verzweigte, gesättigte oder ungesättigte Alkylgruppe mit 6 bis 30 Kohlenstoffatomen, vorzugsweise 8 bis 24 Kohlenstoffatomen und bevorzugt 12 bis 20 Kohlenstoffatomen stehen,
- A für eine lineare oder verzweigte Alkylenrestgruppe mit 2 bis 8 Kohlenstoffatomen steht,
- x und y für Zahlen im Bereich von 1 bis 5000 stehen, vorzugsweise x im Bereich von 10 bis 2000 und insbesondere von 100 bis 1000 liegt, vorzugsweise y im Bereich von 1 bis 100 liegt.

Bevorzugt für das Amodimethicon der Formel (K):

- x im Bereich von 10 bis 2000 und insbesondere von 100 bis 1000 liegt;
- y im Bereich von 1 bis 100 liegt;
- A 3 bis 6 Kohlenstoffatome und insbesondere 4 Kohlenstoffatome umfasst; vorzugsweise A verzweigt ist; bevorzugt A aus den zweiwertigen Resten $-CH_2CH_2CH_2-$ und $-CH_2CH(CH_3)CH_2-$ ausgewählt ist; und
- R1 und R2, die gleich oder verschieden sein können, für einen gesättigten linearen Rest mit 6 bis 30 Kohlenstoffatomen, vorzugsweise 8 bis 24 Kohlenstoffatomen und insbesondere 12 bis 20 Kohlenstoffatomen, beispielsweise eine Dodecyl, Tetradecyl-, Pentadecyl-, Hexadecyl-, Heptadecyl-, Octadecyl-, Nonadecyl- oder Eicosylgruppe, stehen. Vorteilhafterweise R1 und R2 für ein Gemisch von Hexadecyl(Cetyl)- und Octadecyl(Stearyl)-Resten (wobei das Gemisch auch als Cetearyl bekannt ist) stehen;

vorzugsweise das Amodimethicon der Formel (K) Biscetearylamodimethicon ist;
Polyetherdiaminen und insbesondere Polyethylenglykol- und/oder Polypropylenglykol-α,ω-diaminen; Polytetrahydrofuran-α,ω-diaminen (oder Polytetramethylenglykol-α,ω-diaminen) und Polybutadien-α,ω-diaminen;
Polyamidoamin-Dendrimeren mit endständigen Aminfunktionen;
Poly(meth)acrylaten oder Poly(meth)acrylamiden mit seitenständigen primären oder sekundären Aminfunktionen, wie Poly(3-aminopropyl)methacrylamid oder Poly(2-aminoethyl)methacrylat;
und vorzugsweise Polydimethylsiloxanen mit primären Amingruppen am Kettenende oder an Seitenketten;
bevorzugt Polydimethylsiloxanen mit Aminopropyl-Endgruppen am Kettenende;
ausgewählt ist.

**23.** Verfahren nach Anspruch 17, **dadurch gekennzeichnet, dass** es sich bei der zusätzlichen Komponente um eine Aminverbindung handelt, die aus Polydimethylsiloxanen mit primären Amingruppen am Kettenende oder an Seitenketten und 3-Aminopropyltriethoxysilan und vorzugsweise aus Polydimethylsiloxanen mit Aminopropyl-Endgruppen am Kettenende und 3-Aminopropyltriethoxysilan ausgewählt ist.

**24.** Verfahren nach einem der Ansprüche 17 bis 23, **dadurch gekennzeichnet, dass** die Aminverbindung in einem Molverhältnis von Amingruppe der Aminverbindung zu Phosphonsäure des Polymers im Bereich von 0,01 bis 10, vorzugsweise im Bereich von 0,1 bis 5, bevorzugt im Bereich von 0,1 bis 2 und weiter bevorzugt im Bereich von 0,1 bis 1 verwendet wird.

**25.** Verfahren nach Anspruch 17 oder 20, **dadurch gekennzeichnet, dass** dann, wenn die verwendete Zusammensetzung bzw. die verwendeten Zusammensetzungen ein Aminoalkoxysilan enthält bzw. enthalten, sie einen C2-C5-Monoalkohol, vorzugsweise Ethanol oder Isopropanol, insbesondere in einem Gehalt im Bereich von 0,1 bis 5 Gew.-%, bezogen auf das Gesamtgewicht der Zusammensetzung, umfasst bzw. umfassen.

**26.** Verfahren nach Anspruch 17, **dadurch gekennzeichnet, dass** es sich bei der zusätzlichen Komponente um einen Ton handelt, der aus Tonen der Smektit-Familie wie Laponit und Montmorillonit, der Kaolinit-Familie wie Kaolinit, Dickit, Nacrit, gegebenenfalls modifizierten Tonen der Halloysit-, Dombassit-, Antigorit-, Benthierin-, Pyrophyllit-, Montmorillonit-, Beidellit-, Vermiculit-, Talk-, Stevensit-, Hectorit-, Bentonit-, Saponit-, Chlorit-, Sepiolith- und Illit-Familie ausgewählt ist und vorzugsweise aus Kaolinit, Smektiten wie Laponit und Montmorillonit, Bentonit und Saponit ausgewählt ist.

**27.** Verfahren nach Anspruch 17, **dadurch gekennzeichnet, dass** es sich bei der zusätzlichen Komponente um ein Salz von zweiwertigen öder dreiwertigen Metallionen, das aus Salzen von Ionen, die sich von Al(III), Ca(II), Cu(II), Fe(II), Fe(III), Mg(II), Mn(II), Zn(II) und Mischungen davon ableiten, ausgewählt ist und vorzugsweise aus Calciumstearat und Zinkacetat ausgewählt ist, handelt.

**28.** Verfahren nach Anspruch 17, **dadurch gekennzeichnet, dass** es sich bei der zusätzlichen Komponente um ein Metalloxid, das aus Titandioxid, Eisenoxiden, Zirconiumoxiden, zinkoxiden, Ceroxiden und Chromoxiden ausgewählt ist und vorzugsweise aus Eisenoxiden und Titanoxid ausgewählt ist, handelt.

**29.** Verfahren nach einem der Ansprüche 17 bis 28, **dadurch gekennzeichnet, dass** das Mischen der Zusammensetzung, die das Phosphonsäurepolymer umfasst, und der zusätzlichen Komponente oder der Zusammensetzung, die dieselbe enthält, in einer Zeit zwischen 1 Minute und 24 Stunden vor dem Aufbringen der Zusammensetzung auf Keratinmaterialien und vorzugsweise zwischen 5 und 30 Minuten durchgeführt wird.

**30.** Verfahren nach einem der Ansprüche 17 bis 29, **dadurch gekennzeichnet, dass** die auf die Keratinmaterialien aufgebrachte Zusammensetzung ein Öl umfasst.

**31.** Zusammensetzung, erhalten durch Mischen einer Zusammensetzung nach Anspruch 14 oder 15 und einer zusätzlichen Komponente gemäß einem der Ansprüche 17 bis 23 und 26 bis 28 oder einer Zusammensetzung, die dieselbe enthält und ein physiologisch unbedenkliches Medium umfasst.

**32.** Kit, umfassend eine erste Zusammensetzung nach Anspruch 14 oder 15 und eine zweite Zusammensetzung, die

eine zusätzliche Komponente gemäß einem der Ansprüche 17 bis 23 und 26 bis 28 umfasst und ein physiologisch unbedenkliches Medium umfasst, wobei die erste Zusammensetzung und die zweite Zusammensetzung jeweils in einer separaten Verpackungsanordnung verpackt sind.

**33.** Polymer, das durch Umsetzung eines Blockpolymers nach einem der Ansprüche 1 bis 13 mit einer Aminverbindung gemäß einem der Ansprüche 17 bis 24 erhalten wurde, wobei die Umsetzung in wasserfreiem Medium stattfindet, wenn es sich bei der Aminverbindung um ein Aminoalkoxysilan handelt.

## Revendications

**1.** Polymère à blocs comprenant :

au moins un premier bloc ayant une température de transition vitreuse (Tg) supérieure ou égale à 40 °C et obtenu à partir d'au moins un monomère (méth)acrylate ayant la formule $CH_2=C(R_1)-COOR_2$ dans laquelle $R_1$ représente un atome d'hydrogène ou un radical méthyle et $R_2$ représente un groupe cycloalkyle $C_4$-$C_{12}$ ; et au moins un deuxième bloc ayant une température de transition vitreuse (Tg) inférieure ou égale à 20 °C et obtenu à partir d'au moins un monomère acide vinylphosphonique ayant la formule (I) et d'au moins un monomère (méth)acrylate ayant la formule $CH_2=C(R_1)-COOR_3$ dans laquelle $R_1$ représente un atome d' hydrogène ou un radical méthyle et $R_3$ représente un groupe alkyle $C_1$-$C_6$ non substitué soit linéaire, soit ramifié, à l'exception d'un groupe tert-butyle ou d'un groupe méthoxyéthyle ; ledit monomère acide vinylphosphonique ayant la formule (I) étant :

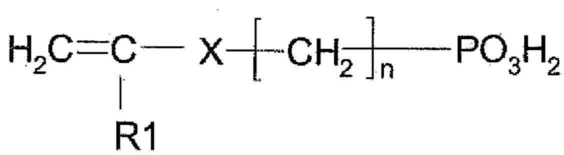

(I)

où :

R1 désigne un atome d'hydrogène ou un groupe $-CH_3$ ;
X désigne une liaison covalente et n désigne un nombre entier de 0 à 14 ;
ou X désigne un groupe -COO- et n désigne un nombre entier de 2 à 6.

**2.** Polymère selon la revendication précédente, **caractérisé en ce que** le premier bloc est obtenu à partir d'au moins un monomère acrylate ayant la formule $CH_2=CH-COOR_2$ dans laquelle $R_2$ représente un groupe cycloalkyle $C_4$-$C_{12}$, et d'au moins un monomère méthacrylate ayant la formule $CH_2=C(CH_3)-COOR'_2$ dans laquelle $R'_2$ représente un groupe cycloalkyle $C_4$-$C_{12}$ ;
et, optionnellement, un monomère additionnel choisi parmi des (méth)acrylates d'alkyle $C_8$-$C_{22}$ linéaires ou ramifiés.

**3.** Polymère selon la revendication précédente, **caractérisé en ce que** pour le premier bloc, ledit monomère acrylate et ledit monomère méthacrylate sont dans des proportions massiques acrylate/méthacrylate de 30/70 à 70/30, préférablement de 40/60 à 60/40, en particulier de 45/55 à 55/45.

**4.** Polymère selon l'une ou l'autre des revendications 2 et 3, **caractérisé en ce que** le premier bloc est obtenu par polymérisation de méthacrylate d'isobornyle et d'acrylate d'isobornyle.

**5.** Polymère selon l'une quelconque des revendications précédentes, **caractérisé en ce que** la proportion du premier bloc est de 60 % à 80 %, et plus préférablement de 65 % à 75 % en poids du polymère.

**6.** Polymère selon l'une quelconque des revendications précédentes, **caractérisé en ce que**, pour le monomère (I) : X désigne une liaison covalente et n est un nombre entier de 0 à 6 ou X désigne un groupe -COO- et n est un nombre entier de 2 à 4.

**7.** Polymère selon l'une des revendications précédentes, **caractérisé en ce que**, pour le monomère (I), R1 représente un atome d'hydrogène et X désigne une liaison covalente et n est un nombre entier de 0 à 4.

8. Polymère selon l'une des revendications précédentes, **caractérisé en ce que** le monomère (I) est choisi parmi :

l'acide vinylphosphonique ;
l'acide 3-buténylphosphonique ;
l'acide 4-penténylphosphonique ;
l'acide 10-undécénylphosphonique ;
l'acide 11-dodécénylphosphonique ;
l'ester 2-phosphonoéthylique de l'acide 2-méthyl-2-propénoïque ;
l'ester 2-phosphonoéthylique de l'acide 2-propénoïque ;
et préférablement l'acide vinylphosphonique.

9. Polymère selon l'une des revendications précédentes, **caractérisé en ce que** le deuxième bloc comprend un monomère choisi parmi l'acrylate d'isobutyle, l'acrylate d'éthyle, l'acrylate de n-butyle et l'acrylate de méthoxyéthyle, ou des mélanges de ceux-ci, et préférablement l'acrylate d'isobutyle.

10. Polymère selon l'une quelconque des revendications précédentes, **caractérisé en ce que**, pour le deuxième bloc, le monomère acide vinylphosphonique (I) et ledit monomère (méth) acrylate sont dans des proportions massiques (méth)acrylate/acide vinylphosphonique (I) de 1 à 10, préférentiellement de 2 à 9, en particulier de 3 à 8, ou autrement de 4 à 7.

11. Polymère selon l'une des revendications précédentes, **caractérisé en ce que** la proportion du deuxième bloc est de 20 % à 40 % et plus préférablement de 25 % à 35 % en poids du polymère.

12. Polymère selon l'une des revendications précédentes, **caractérisé en ce qu'**il comprend un segment intermédiaire comprenant au moins un monomère constitutif du premier bloc et au moins un monomère constitutif du deuxième bloc, et est en particulier un polymère statistique.

13. Polymère selon l'une des revendications précédentes, **caractérisé en ce qu'**il a un indice de polydispersité supérieur à 2, préférablement de 3 à 20.

14. Composition comprenant, dans un milieu physiologiquement acceptable, un polymère à blocs selon l'une des revendications 1 à 13.

15. Composition selon la revendication précédente, **caractérisée en ce que** le polymère à blocs est présent dans une quantité de 0,1 % à 40 % en poids, relativement au poids total de la composition dérivée du mélange extemporané, préférablement de 0,5 % à 35 % en poids, préférentiellement de 1 % à 30 % en poids, et plus préférentiellement de 10 % à 30 % en poids.

16. Procédé cosmétique pour le traitement de matières kératiniques, comprenant l'application topique sur les matières kératiniques d'une composition selon la revendication 14 ou 15.

17. Procédé selon la revendication précédente, **caractérisé en ce qu'**une composition obtenue en mélangeant la composition selon l'une ou l'autre des revendications 14 et 15 et un constituant additionnel choisi parmi :

(i) un composé amine choisi parmi des composés polyamines portant plusieurs groupes amine primaire et/ou groupes amine secondaire et des aminoalcoxysilanes,
(ii) des sels d'ions métalliques divalents ou trivalents,
(iii) des argiles,
(iv) des oxydes métalliques,

ou une composition contenant les mêmes constituants et comprenant un milieu physiologiquement acceptable, est appliquée topiquement sur les matières kératiniques, la/les composition(s) utilisée(s) étant anhydre(s) lorsque le constituant additionnel est un aminoalcoxysilane.

18. Procédé selon la revendication précédente, **caractérisé en ce que** le composé polyamine comprend de 2 à 20 atomes de carbone.

19. Procédé selon l'une ou l'autre des revendications 17 et 18, **caractérisé en ce que** le composé polyamine est choisi

parmi le N-méthyl-1,3-diaminopropane, le N-propyl-1,3-diaminopropane, le N-isopropyl-1,3-diaminopropane, le N-cyclohexyl-1,3-diaminopropane, le 2-(3-aminopropylamino)éthanol, la 3-(2-aminoéthyl)aminopropylamine, la bis(3-aminopropyl)amine, la méthylbis(3-aminopropyl)aminé, le N-(3-aminopropyl)-1,4-diaminobutane, la N,N-diméthyldipropylènetriamine, le 1,2-bis(3-aminopropylamino)éthane, la N,N'-bis(3-aminopropyl)-1,3-propanediamine, l'éthylènediamine, la 1,3-propylènediamine, la 1,4-butylènediamine, la lysine, la cystamine, la xylènediamine, la tris(2-aminoéthyl)amine et la spermidine.

**20.** Procédé selon la revendication 17, **caractérisé en ce que** l'aminoalcoxysilane a la formule (III) :

$$R'_1Si(OR'_2)_z(R'_3)_x \qquad (III)$$

dans laquelle :

- $R'_1$ est une chaîne hydrocarbonée $C_1$-$C_6$ linéaire ou ramifiée, saturée ou insaturée, cyclique ou acyclique substituée avec un groupe choisi parmi les groupes suivants :

  - un groupe amine $NH_2$ ou NHR où R est un groupe alkyle $C_1$-$C_4$,
  - un groupe aryle ou aryloxy substitué avec un groupe amino ou avec un groupe aminoalkyle $C_1$-$C_4$,

  la chaîne de $R'_1$ étant éventuellement interrompue avec un hétéroatome (O, S, NH) ou un groupe carbonyle (CO), $R'_1$ étant lié à l'atome de silicium directement via un atome de carbone,

- $R'_2$ et $R'_3$, qui peuvent être identiques ou différents, représentent un groupe alkyle linéaire ou ramifié comprenant de 1 à 6 atomes de carbone,
- z désigne un nombre entier de 1 à 3, et
- x désigne un nombre entier de 0 à 2,

où z + x = 3 ;
et est préférablement le 3-aminopropyltriéthoxysilane.

**21.** Procédé selon la revendication 17, **caractérisé en ce que** le composé polyamine est choisi parmi des polymères à base d'amines, en particulier ayant une masse moléculaire moyenne en poids de 500 à 1 000 000, préférablement de 500 à 500 000, et préférentiellement de 500 à 100 000.

**22.** Procédé selon la revendication précédente, **caractérisé en ce que** le composé polyamine est un polymère à base d'amines choisi parmi des poly(alkylèneimines $C_2$-$C_5$), et en particulier des polyéthylèneimines et des polypropylèneimines, en particulier des poly(éthylèneimine)s ; la poly(allylamine) ; des polyvinylamines et des copolymères de celles-ci, en particulier avec des vinylamides ; des copolymères de vinylamine/vinylformamide ; des acides polyaminés portant des groupes $NH_2$, tels que la polylysine ; l'aminodextrane ; un alcool amino-polyvinylique ; des copolymères à base d'acrylamidopropylamine ; des chitosanes ;
des polydiméthylsiloxanes comprenant des groupes amine primaire à l'extrémité de la chaîne ou sur des chaînes latérales, par exemple des groupes aminopropyle latéraux ou terminaux, par exemple ceux ayant la formule (A) ou (B) ou (C) :

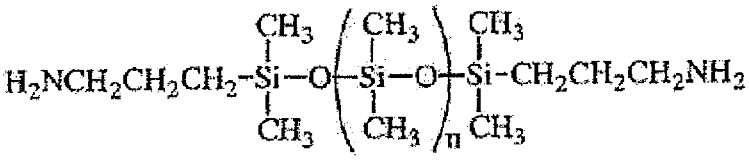

(A)

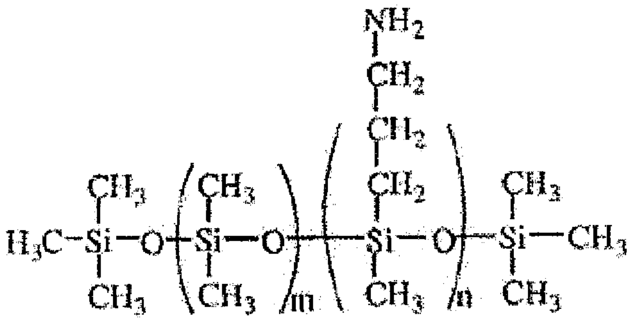

(B)

$$H_2NCH_2CH_2CH_2\text{-}Si(CH_3)_2\text{-}O\text{-}[Si(CH_3)_2\text{-}O]_n\text{-}Si(CH_3)_2C_4H_9 \qquad (C)$$

où :

dans la formule (A), la valeur de n est telle que la masse moléculaire moyenne en poids de la silicone est de 500 à 55 000 ;
dans la formule (B), les valeurs de n et m sont telles que la masse moléculaire moyenne en poids de la silicone est de 1 000 à 55 000 ;
dans la formule (C), la valeur de n est telle que la masse moléculaire moyenne en poids de la silicone est de 500 à 3 000 ;

les amodiméthicones ayant la formule (D) :

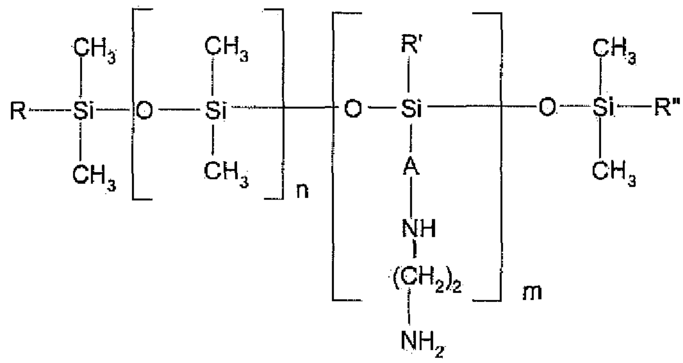

(D)

dans laquelle R, R' et R", qui peuvent être identiques ou différents, représentent chacun un groupe alkyle ou hydroxyle $C_1$-$C_4$, A représente un groupe alkylène $C_3$, et m et n sont tels que la masse moléculaire moyenne en poids du composé est de 5 000 à 500 000 environ ;
les amodiméthicones ayant la formule (K) :

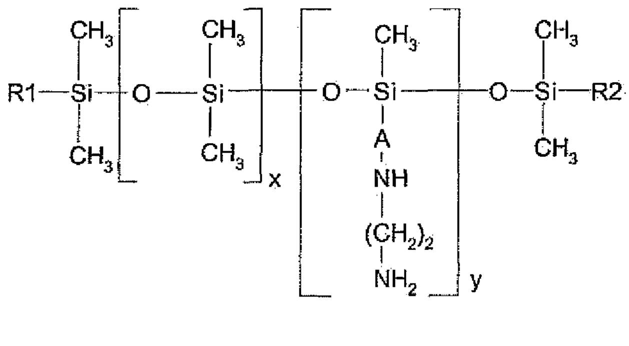

(K)

dans laquelle :

- R1 et R2, qui peuvent être identiques ou différents, préférablement identiques, représentent un groupe alkyle linéaire ou ramifié, saturé ou insaturé, comprenant de 6 à 30 atomes de carbone, préférablement de 8 à 24 atomes de carbone, et préférentiellement de 12 à 20 atomes de carbone,
- A représente un groupe radical alkylène linéaire ou ramifié contenant de 2 à 8 atomes de carbone,
- x et y sont des nombres de 1 à 5 000 ; préférablement, x vaut de 10 à 2 000, et en particulier de 100 à 1 000 ; préférablement, y vaut de 1 à 100.

Préférentiellement, pour l'amodiméthicone ayant la formule (K) :

- x vaut de 10 à 2 000, et en particulier de 100 à 1 000 ;
- y vaut de 1 à 100 ;
- A comprend de 3 à 6 atomes de carbone, et en particulier 4 atomes de carbone ; préférablement, A est ramifié ; préférentiellement, A est choisi parmi les radicaux divalents : $-CH_2CH_2CH_2-$ et $-CH_2CH(CH_3)CH_2-$ ; et
- R1 et R2, qui peuvent être identiques ou différents, représentent un radical linéaire saturé comprenant de 6 à 30 atomes de carbone, préférablement de 8 à 24 atomes de carbone, et en particulier de 12 à 20 atomes de carbone, par exemple un groupe dodécyle, tétradécyle, pentadécyle, hexadécyle, heptadécyle, octadécyle, nonadécyle ou éicosyle. R1 et R2 représentent avantageusement un mélange de radicaux hexadécyle (cétyle) et octadécyle (stéaryle) (mélange également connu sous le nom de cétéaryle) ;

préférablement, l'amodiméthicone ayant la formule (K) est la bis-cétéarylamodiméthicone ;
des polyétherdiamines, et en particulier le polyéthylène glycol et/ou le polypropylène glycol $\alpha,\omega$-diamines ; le polytétrahydrofurane (ou polytétraméthylène glycol) $\alpha,\omega$-diamines, et le polybutadiène $\alpha,\omega$-diamines ;
des dendrimères polyamidoamines portant des fonctions terminales amine ;
des poly(méth)acrylates ou des poly(méth)acrylamides portant des fonctions latérales amine primaire ou amine secondaire, tels que le poly(3-aminopropyl)méthacrylamide ou le méthacrylate de poly(2-aminoéthyle) ;
et préférablement des polydiméthylsiloxanes comprenant des groupes amine primaire à l'extrémité de la chaîne ou sur des chaînes latérales ;
préférentiellement, des polydiméthylsiloxanes comprenant des groupes terminaux aminopropyle à l'extrémité de la chaîne.

**23.** Procédé selon la revendication 17, **caractérisé en ce que** le constituant additionnel est un composé amine choisi parmi des polydiméthylsiloxanes comprenant des groupes amine primaire à l'extrémité de la chaîne ou sur des chaînes latérales et le 3-aminopropyltriéthoxysilane ; et préférablement parmi des polydiméthylsiloxanes comprenant des groupes terminaux aminopropyle à l'extrémité de la chaîne et le 3-aminopropyltriéthoxysilane.

**24.** Procédé selon l'une quelconque des revendications 17 à 23, **caractérisé en ce que** le composé amine est utilisé selon un rapport molaire du groupe amine du composé amine/acide phosphonique du polymère de 0,01 à 10, préférablement de 0,1 à 5, préférentiellement de 0,1 à 2, et plus préférentiellement de 0,1 à 1.

**25.** Procédé selon l'une ou l'autre des revendications 17 et 20, **caractérisé en ce que** lorsque la/les composition(s) utilisée(s) contien(nen)t un aminoalcoxysilane, elle(s) comprend/comprennent un monoalcool $C_2$-$C_5$, préférablement l'éthanol ou l'isopropanol, en particulier dans une quantité de 0, 1 % à 5 % en poids, relativement au poids total de la composition.

**26.** Procédé selon la revendication 17, **caractérisé en ce que** le constituant additionnel est une argile choisie parmi des argiles de la famille des smectites, telles que la laponite et la montmorillonite, de la famille des kaolinites, telles que la kaolinite, la dickite, la nacrite, des argiles optionnellement modifiées de la famille de l'halloysite, de la dombassite, de l'antigorite, de la berthiérine, de la pyrophyllite, de la montmorillonite, de la beidellite, de la vermiculite, du talc, de la stevensite, de l'hectorite, de la bentonite, de la saponite, de la chlorite, de la sépipiolite et de l'illite ; et préférablement choisie parmi la kaolinite, des smectites telles que la laponite et la montmorillonite, la bentonite et la saponite.

**27.** Procédé selon la revendication 17, **caractérisé en ce que** le constituant additionnel est un sel d'ions métalliques divalents ou trivalents choisis parmi des sels d'ions dérivés de Al(III), Ca(II), Cu(II), Fe(II), Fe(III), Mg(II), Mn(II), Zn(II) et de mélanges de ceux-ci ; préférablement choisi parmi le stéarate de calcium et l'acétate de zinc.

**28.** Procédé selon la revendication 17, **caractérisé en ce que** le constituant additionnel est un oxyde de métal choisi parmi le dioxyde de titane, des oxydes de fer, des oxydes de zirconium, des oxydes de zinc, des oxydes de cérium

et des oxydes de chrome ; préférablement choisi parmi des oxydes de fer et le dioxyde de titane.

**29.** Procédé selon l'une quelconque des revendications 17 à 28, **caractérisé en ce que** le mélange de la composition comprenant le polymère phosphonique et le constituant additionnel, ou de la composition contenant les mêmes constituants, est effectué durant une période de 1 minute à 24 heures avant l'application de celle-ci sur les matières kératiniques, et préférablement de 5 à 30 minutes.

**30.** Procédé selon l'une quelconque des revendications 17 à 29, **caractérisé en ce que** la composition appliquée sur les matières kératiniques comprend une huile.

**31.** Composition obtenue en mélangeant une composition selon la revendication 14 ou 15 et un constituant additionnel tel que défini dans l'une des revendications 17 à 23 et 26 à 28, ou une composition contenant les mêmes constituants et comprenant un milieu physiologiquement acceptable.

**32.** Kit comprenant une première composition selon la revendication 14 ou 15 et une deuxième composition comprenant un composé additionnel tel que défini dans l'une des revendications 17 à 23 et 26 à 28 et comprenant un milieu physiologiquement acceptable, les première et deuxième compositions étant chacune emballée dans un ensemble emballage distinct.

**33.** Polymère obtenu en faisant réagir un polymère à blocs selon l'une des revendications 1 à 13 avec un composé amine tel que défini selon l'une des revendications 17 à 24, la réaction ayant lieu dans un milieu anhydre lorsque le composé aminé est un aminoalcoxysilane.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 1882709 A
- WO 2008155059 A


### Non-patent literature cited in the description


- Polymer Handbook. John Wiley, 1989
- Mineralogie des argiles. **S. CAILLÈRE ; S. HÉNIN ; M. RAUTUREAU.** Mineralogy of Clays. 1982